# EUROPÄISCHE PATENTSCHRIFT

(11) **EP 3 334 531 B1**
(45) Veröffentlichungstag und Bekanntmachung des Hinweises auf die Patenterteilung: **22.07.2020**
(21) Anmeldenummer: 16753568.1
(22) Anmeldetag: 09.08.2016
(51) Int. Cl.: B01L 3/00, C12M 3/06

(54) **VORRICHTUNG UND VERFAHREN ZUR BESTIMMUNG DER MIGRATIONSFÄHIGKEIT AMÖBOID BEWEGLICHER ZELLEN**
MIGRATION DEVICE AND METHOD FOR OPERATING A MIGRATION DEVICE
DISPOSITIF DE MIGRATION ET PROCÉDÉ DE FONCTIONNEMENT D'UN DISPOSITIF DE MIGRATION

(30) Priorität: 12.08.2015 EP 15180818
(43) Veröffentlichungstag der Anmeldung: 20.06.2018
(73) Patentinhaber: Meon Medical Solutions GmbH & Co. KG, 8010 Graz (AT)
(72) Erfinder: HUEMER, Herfried, 8330 Feldbach (AT); ZAHRL, Doris, 8020 Graz (AT); RÜTHER, Horst, 8047 Hart / Graz (AT)
(74) Vertreter: Babeluk, Michael
(86) Internationale Anmeldenummer: PCT/AT2016/050252
(87) Internationale Veröffentlichungsnummer: WO 2017/024328

(56) Entgegenhaltungen:
- WO-A1-2015/032889
- US-A1- 2001 004 530
- D. J. BARRY ET AL: "Nitrocellulose as a General Tool for Fungal Slide Mounts", JOURNAL OF CLINICAL MICROBIOLOGY, Bd. 45, Nr. 3, 1. März 2007 (2007-03-01), Seiten 1074-1075, XP055250510, US ISSN: 0095-1137, DOI: 10.1128/JCM.01609-06

## Beschreibung

Die Erfindung betrifft die Bestimmung der Migrationsfähigkeit amöboid beweglicher Zellen. Insbesondere betrifft die Erfindung eine Migrationsvorrichtung zur Bestimmung der Migrationsfähigkeit amöboid beweglicher Zellen, die Verwendung einer Migrationsvorrichtung zum Bestimmen der Migrationsfähigkeit amöboid beweglicher Zellen sowie ein Verfahren zum Betreiben einer Migrationsvorrichtung.

### Technischer Hintergrund:

Die Migrationsfähigkeit amöboid beweglicher Zellen kann bestimmt werden, indem die natürlichen Verhältnisse im Körper unter standardisierten Bedingungen außerhalb des Körpers, in vitro, simuliert werden. Dabei ersetzt ein poröser Membranfilter bzw. eine Migrationsmatrix bestimmter Porenweite und Dicke das Gewebe des Körpers und stellen ein künstliches Hindernis dar, in welche z.B. Neutrophile eindringen können. Auf diese Weise kann die Bereitschaft der Neutrophilen beurteilt werden, unter standardisierten Bedingungen (Filterqualität, Milieu, Zeit, Reizstoffe) in Filter einzudringen. Die Zahl und die Verteilung der in den Filter eingedrungenen Neutrophilen kann dabei gemessen und bewertet werden.

Ein Membranfiltersystem zur Messung der Zellmigration ist in der AT 394 455 B beschrieben. Darin ist ein System gezeigt, das aus einem Membranfilter besteht, an welchem einseitig eine Trägermatrix für Testsubstanzen angebracht ist, die wiederum an der dem Membranfilter gegenüber liegenden Seite von einer undurchlässigen Grenzschicht abgeschlossen wird. Die festen Testsubstanzen lösen sich in dem wässrigen Milieu, in dem die zu untersuchenden Zellen von der Membranfilterseite an das System herangebracht werden, wobei das Lösungsverhalten durch Liberationsstabilisatoren beeinflusst werden kann. Die undurchlässige Grenzschicht zwingt die gelösten Testsubstanzen, in Richtung des Membranfilters zu diffundieren, um hier die Zellmigration zu beeinflussen. Die Beurteilung der Migrationsleistung geschieht anhand der Zahl und Verteilung der Zellen im Membranfilter, die mikroskopisch bestimmt werden.

Zur Migrationsmessung in vitro wird weiterhin ein Behälterring an der Membranfilterseite des Systems befestigt. In den Behälterring wird eine Suspension der zu testenden Zellen eingefüllt. Um anschließend die Migrationsleistung beurteilen zu können, ist es erforderlich, dass der Behälterring vom Membranfiltersystem entfernt wird und dass das Membranfiltersystem für die Untersuchung auf einem Objektträger befestigt wird.

In der AT 406 310 B sowie in der US 2001/0004530 A1 ist eine ähnliche Vorrichtung zur Messung der Migrationsfähigkeit von amöboid beweglichen Zellen beschrieben. Die Vorrichtung ist so ausgeführt dass die Fläche des Membranfilters mindestens 1,6-mal so groß ist wie die Fläche der Bodenöffnung des Behälterrings. Im montierten Zustand verbleibt so eine ausreichende Porosität des zwischen der unteren Stirnfläche des Behälterrings und der Bodenfläche anliegenden Bereichs des Membranfilters, so dass nach Einfüllen in den Behälterring ein Teil der nicht zellulären Flüssigkeitsphase in den außerhalb der Bodenfläche des Behälters befindlichen Bereich des Membranfilters einfließt. Dadurch werden die Zellen mit dem Membranfilter rascher in Kontakt gebracht.

In der WO 2015/032 889 A1 wird eine Vorrichtung zur In-Vitro-Modellierung von lebenden Geweben und Organen beschrieben, wobei es hier im Wesentlichen darum geht, dass auf "culturing membranes" Zellen aufgebracht und dort kultiviert werden (beispielsweise Lungenzellen), um damit Untersuchungen und Studien durchzuführen. Die sogenannten "culturing membranes" sind zwischen Kammern (access chambers bzw. culturing chamber) angeordnet, die über Zu- und Ablaufkanälen mit den für die Untersuchungen benötigten Substanzen beschickt werden können. Die Untersuchung der Einwanderung von Zellen in eine poröse Membran hinein ist mit einer Vorrichtung gemäß WO 2015/032 889 A1 nicht durchführbar.

Aus D. J. BARRY et al: "Nitrocellulose as a General Tool for Fungal Slide Mounts", Journal of Clinical Microbiology, Bd. 45, Nr. 3, 1. März 2007 ist es bekannt, in Rahmen eingespannte Membranen aus Nitrozellulose zu verwenden, um in situ das Wachstum von Pilzkulturen unter dem Mikroskop zu untersuchen. Nach einer Wachstumsphase werden die Membranen gefärbt mit Immersionsöl transparent gemacht und unter dem Mikroskop untersucht.

### Definitionen:

- **Migration:** Unter Migration bzw. Zellmigration (Latein: *migrare,* 'wandern') versteht man die aktive Ortsveränderung (Lokomotion) von Zellen oder Zellverbänden. Der Überbegriff "Migration" schließt die ungerichtete Spontanbewegung (*random migration*), die gerichtete, chemotaktische Bewegung und die Änderung der Bewegungsgeschwindigkeit (Chemokinetik) ein.
- **Migrationsmatrix:** Im Kontext dieser Anmeldung ist unter Migrationsmatrix eine dünne, vorzugsweise offenporige Schicht zu verstehen, in welche amöboid bewegliche Zellen eindringen (migrieren) können, wobei die mittlere Porengröße der Migrationsmatrix bevorzugt kleiner ist als der mittlere Durchmesser der amöboid beweglichen Zellen.
- **amöboid bewegliche Zellen:** Die amöboide Bewegung beschreibt eine Kriechbewegung von Zellen, wie z. B. bei Amöben, bestimmte Leukozyten, Amöbenalgen und manchen Krebszellen.

### Zusammenfassung der Erfindung:

Die Aufgabe der Erfindung kann darin gesehen werden, das Bestimmen der Migrationsfähigkeit amöboid beweglicher Zellen zu vereinfachen.

Diese Aufgabe wird durch die Gegenstände der unabhängigen Ansprüche gelöst. Weiterbildungen und Ausführungsformen sind den abhängigen Ansprüchen, der Beschreibung und den Figuren zu entnehmen.

Ein erster Aspekt der Erfindung betrifft eine Migrationsvorrichtung zur Bestimmung der Migrationsfähigkeit amöboid beweglicher Zellen. Die Migrationsvorrichtung weist eine Probenkammer, mindestens eine in der Probenkammer angeordnete Migrationsmatrix, mindestens einen Fluidauslass und mindestens eine Abführungsstruktur auf. Die Abführungsstruktur ist zum Abführen eines Fluides aus der Probenkammer und/oder aus der Migrationsmatrix ausgeführt.

Ein Kerngedanke der Erfindung kann darin gesehen werden, dass die Migrationsvorrichtung eine Struktur (Abführungsstruktur) aufweist, die dazu ausgeführt ist, um ein Fluid aus der Probenkammer heraus zu leiten. Auf diese Weise kann das Benutzen bzw. das Betreiben der Migrationsvorrichtung zur Bestimmung der Migrationsfähigkeit amöboid beweglicher Zellen vereinfacht werden. Beispielsweise können mittels der Abführungsstruktur verschiedene Flüssigkeiten und/oder Gase (d.h. Fluide) aus der Probenkammer abgeleitet werden, so dass das Entfernen dieser Fluide aus der Probenkammer mittels einer Pipette o.Ä. nicht erforderlich ist. Dies ermöglicht wiederum eine automatisiertere Bestimmung der Migrationsfähigkeit mittels der Migrationsvorrichtung. Weiterhin kann die Abführungsstruktur dazu ausgeführt sein, um die Bildung von Luftblasen während des Befüllens der Probenkammer zu verhindern. Ferner kann die Abführungsstruktur auch dazu verwendet werden, um Luft durch die Probenkammer zu saugen, um einen Trocknungsprozess herbeizuführen. Alle diese Prozesse, die mittels der Abführungsstruktur durchgeführt werden, vereinfachen das Bestimmen der Migrationsfähigkeit amöboid beweglicher Zellen mittels der Migrationsvorrichtung. Mit anderen Worten kann aufgrund der Abführungsstruktur die Notwendigkeit von manuellen Arbeitsschritten beim Betreiben der Migrationsvorrichtung entfallen bzw. das Betreiben der Migrationsvorrichtung kann auf einige wenige einfache, unkritische Handgriffe beschränkt werden. Weitere Vorteile und Anwendungen der erfindungsgemäßen Migrationsvorrichtung werden im Kontext detaillierter Ausführungsbeispiele beschrieben werden.

Viele eukaryotische Zellen, wie z.B. Fibroblasten, Keratinozyten, Neuronen, Immunzellen, und Amöben sind durch amöboide Bewegung bzw. aktive Lokomotion zur Migration befähigt. Unter "Migration" bzw. "Zellmigration" bzw. "Zellwanderung" kann im Kontext dieser Erfindung die aktive Ortsveränderung amöboid beweglicher biologischer Zellen oder Zellverbände verstanden werden. Der Überbegriff "Migration" kann jegliche aktive amöboide Zellbewegung, wie die ungerichtete aktive Spontanbewegung (random migration), die gerichtete chemotaktische Bewegung (Chemotaxis) und die Änderung der aktiven Bewegungsgeschwindigkeit (Chemokinetik) einschließen. Unter "Migrationsleistung" bzw. "Migrationsfähigkeit" bzw. "Migrationsaktivität" kann im Kontext dieser Erfindung die aktive Fortbewegung bzw. die Fähigkeit amöboid beweglicher Zellen zur aktiven Fortbewegung verstanden werden.

Unter einer Migrationsvorrichtung kann im Kontext der Erfindung eine Vorrichtung verstanden werden, welche zur Bestimmung der Migrationsfähigkeit amöboid beweglicher Zellen verwendet werden kann. Die Migrationsvorrichtung kann so ausgebildet sein, dass sie sowohl für die Mikroskopie mit einem für die Zellbiologie geeigneten Standard-Inversmikroskop und/oder für automatisierbare Manipulationsschritte, z.B. Temperieren, Waschen, Färben, ausgeführt ist.

Grundsätzlich kann mit der erfindungsgemäßen Vorrichtung die Migrationsfähigkeit jeder zur amöboiden Fortbewegung fähigen biologischen Zelle oder von Zellverbänden bestimmt werden. Bevorzugt kann die erfindungsgemäße Migrationsvorrichtung zur Bestimmung der Migrationsfähigkeit weißer Blutzellen (Leukozyten), wie z.B. von Monozyten/Makrophagen oder von neutrophilen Granulozyten (Neutrophilen) verwendet werden. Besonders bevorzugt kann die erfindungsgemäße Migrationsvorrichtung zur in-vitro Bestimmung der Migrationsfähigkeit polymorphkerniger neutrophiler Granulozyten (PMN) aus Vollblut oder aus verdünntem Vollblut als Probe verwendet werden.

Im Kontext der Erfindung kann unter der Bestimmung der Migrationsfähigkeit die die in-vitro Migration, Messung und/oder Bewertung der Migration bzw. der Nicht-Migration amöboid beweglicher Zellen verstanden werden. Dabei kann die Migration bevorzugt durch Einwandern in einen porösen Membranfilter bzw. in eine Migrationsmatrix erfolgen, wonach nach Fixieren und Färben der eingewanderten Zellen die Verteilung der Zellen in und auf dem Membranfilter bzw. das Migrationsprofil mikroskopisch vermessen und bewertet werden kann.

Die Migrationsvorrichtung weist gemäß der Erfindung ein Gehäuse auf, welches zumindest teilweise aus Kunststoff gefertigt sein kann. Dieses Gehäuse kann die Probenkammer, den Fluidauslass sowie die Abführungsstruktur enthalten. Mit anderen Worten können die Probenkammer, der Fluidauslass und die Abführungsstruktur in der Migrationsvorrichtung bzw. im Gehäuse der Migrationsvorrichtung ausgebildet sein.

Das Gehäuse der Migrationsvorrichtung kann dabei einteilig oder aber auch mehrteilig, zum Beispiel zweiteilig, ausgeführt sein. Beispielsweise kann das Gehäuse der Migrationsvorrichtung ein Oberteil und ein Unterteil aufweisen, die zusammenfügbar bzw. zusammensetzbar sind. Das Gehäuse bzw. das Oberteil und das Unterteil können mittels Spritzgussverfahren gefertigt sein. Das Oberteil und das Unterteil können jeweils einstückig ausgebildet sein.

Unter der Probenkammer kann im Kontext der Erfindung eine Vertiefung bzw. Ausnehmung, die in der Migrationsvorrichtung bzw. im Gehäuse der Migrationsvorrichtung ausgebildet ist, verstanden werden. Beispielsweise kann die Probenkammer eine in der Migrationsvorrichtung bzw. im Gehäuse ausgebildete Vertiefung bzw. Ausnehmung mit kreisförmigem Querschnitt sein. Jedoch kann der Querschnitt der Probenkammer auch eine andere Form aufweisen, z.B. eine ovale oder rechteckige Form. Weiterhin kann die Probenkammer eine Seitenwand und eine Bodenfläche aufweisen, auf welche im Folgenden weiter eingegangen wird. Die Probenkammer kann dazu ausgeführt sein, um darin die Bestimmung der Migrationsfähigkeit amöboid beweglicher Zellen durchzuführen. Beispielsweise kann eine Probenflüssigkeit in die Probenkammer eingebracht werden, um die Migrationsfähigkeit der in der Probenflüssigkeit enthaltenen amöboid beweglichen Zellen zu bestimmen.

Die Probenkammer kann eine Eingangsöffnung aufweisen, durch welche Fluide in die Probenkammer eingeführt werden können. Diese Eingangsöffnung kann verschließbar sein. Beispielsweise kann die Migrationsvorrichtung einen Verschluss, z.B. eine Abdeckung, zum Verschließen der Eingangsöffnung der Probenkammer aufweisen.

Unter der Matrix kann im Kontext der vorliegenden Erfindung auch ein Membranfilter verstanden werden. Eine solche Matrix bzw. ein solcher Membranfilter, der in einer Migrationsvorrichtung gemäß der vorliegenden Erfindung eingesetzt werden und Teil der Migrationsvorrichtung sein kann, ist beispielsweise in der AT 394 455 B beschrieben. Der Membranfilter kann die migrierenden Zellen aufnehmen. Er kann bevorzugt Kohlenhydratverbindungen wie Zellulosederivaten (Acetat, Nitrat), Polycarbonat, oder aus Kunststoffen wie Polyamid oder Polyvinylchlorid enthalten. Die Filter können ohne weitere Präparation verwendet werden oder die Innenwandungen ihrer Poren können mit Substanzen beschichtet werden, zu denen die wandernden Zellen gewisse Affinitäten aufweisen, wie Kollagen, Fibrin, Heteroglycane, oder Kohlenhydratpolymere wie Agar. Die Porenweite des Membranfilters richtet sich nach dem untersuchten Zelltyp und der Aufgabenstellung der Untersuchung. Im Falle von Leukozyten haben sich Porenweiten zwischen 1 µm und 8 µm als vorteilhaft erwiesen. Diese Angabe ist nicht als limitierendes Merkmal der Erfindung zu verstehen, da je nach Zelltypen andere Porenweiten günstiger sein können.

Ferner kann die Matrix auch mehrere Matrices, das heißt Matrizen, umfassen. Beispielsweise kann die Matrix zwei schichtartig übereinander angeordnete erste und zweite Matrices aufweisen. Die erste Matrix kann beispielsweise eine Migrationsmatrix und die zweite Matrix kann beispielsweise eine Depotmatrix sein.

Die zweite Matrix bzw. Depotmatrix kann im Kontext der vorliegenden Erfindung auch ein poröser Membranfilter sein, der einen oder mehrere Wirkstoffe enthält. Die Depotmatrix kann ein poröses Material, in das ein Wirkstoff in festem Aggregatzustand eingelagert werden kann und der sich bei Kontakt mit einer Probenflüssigkeit auflöst, aufweisen.

Die Matrix liegt gemäß der Erfindung auf einer Bodenfläche der Probenkammer auf. Im Falle, dass die Matrix eine Depotmatrix und eine Migrationsmatrix aufweist, können die Depotmatrix auf der Bodenfläche der Probenkammer und die Migrationsmatrix auf der Depotmatrix angeordnet sein. Mit anderen Worten können von oben nach unten gesehen, die Migrationsmatrix auf der Depotmatrix und die Depotmatrix auf der Bodenfläche der Probenkammer aufliegen. Die Depotmatrix kann einen Wirkstoff enthalten, wobei als Wirkstoff alle Substanzen verstanden werden können, welche auf die Migration von amöboid beweglichen Zellen eine fördernde oder hemmende Wirkung ausüben können. Auch andere Wirkstoffe können enthalten sein. Je nach Aufgabenstellung kann die Depotmatrix auch keinen Wirkstoff enthalten oder auch ganz entfallen.

Als Depotmatrix kann ein poröser Membranfilter aus Zellulosenitrat eingesetzt werden und als Wirkstoff kann das chemotaktische Tripeptid N-Formyl-Metyonyl-Leucyl-Phenylalanin (FMLP), das beispielsweise mit Agar in die Depotmatrix eingebracht ist, verwendet werden. Eine völlig analog aufgebaute Depotmatrix ohne Wirkstoff kann als Leerkontrolle dienen.

Unter dem Fluidauslass kann z.B. eine in der Migrationsvorrichtung bzw. im Gehäuses der Migrationsvorrichtung ausgebildete Öffnung verstanden werden, durch welche ein Fluid aus der Probenkammer ausgelassen werden kann. Dabei kann die Migrationsvorrichtung einen oder mehrere (zum Beispiel zwei) voneinander getrennte Fluidauslässe aufweisen. Der Fluidauslass kann derart ausgebildet sein, dass eine Absaugpumpe bzw. eine Vakuumpumpe an den Fluidauslass anschließbar ist, so dass Fluide aus der Probenkammer abgepumpt werden können und/oder ein Unterdruck in der Abführungsstruktur erzeugt werden kann. Beispielsweise kann der Fluidauslass eine Art Absaugnippel, z.B. ein Luer, aufweisen, an welchen die Absaugpumpe bzw. die Vakuumpumpe anschließbar ist. Sowohl Gase, z.B. Luft, als auch Flüssigkeiten, z.B. Wasser, Blut, etc., sowie auch Mischungen aus Gas und Flüssigkeiten können im Kontext der Erfindung als Fluide verstanden werden.

Unter der Abführungsstruktur kann eine in der Migrationsvorrichtung bzw. im Gehäuse der Migrationsvorrichtung vorgesehene Struktur verstanden werden, die zum Abführen eines Fluides aus der Probenkammer und/oder aus der oder durch die Migrationsmatrix zum Fluidauslass ausgeführt ist. Diese Abführungsstruktur kann dabei direkt in der Migrationsvorrichtung bzw. im Gehäuse ausgebildet sein, z.B. indem eine oder mehrere Ausnehmungen in der Migrationsvorrichtung bzw. im Gehäuse ausgebildet sind. Die Abführungsstruktur kann dabei eine oder mehrere mechanische Unterstrukturen aufweisen. Zum Beispiel kann die Abführungsstruktur zusätzliche Komponenten oder Elemente umfassen, die an der Migrationsvorrichtung bzw. am Gehäuse angebracht und/oder befestigt sind, z.B. zusätzliche Röhrchen bzw. Kanäle. Weiterhin kann die Abführungsstruktur eine an der Probenkammer angeordnete Abflussstruktur umfassen, z.B. eine Öffnung, eine Nut, ein verzweigtes Nutsystem, eine Gitterstruktur oder eine andere mechanische Struktur. Im Kontext der vorliegenden Erfindung wird der Begriff "Abführungsstruktur" als Oberbegriff für verschiedene Ausführungsformen der fluidmechanischen Verbindung zwischen der Probenkammer und dem Fluidauslass verwendet. Eine Abführungsstruktur stellt daher eine fluidmechanische Verbindung zwischen der Probenkammer und dem Fluidauslass bereit. Die Abführungsstruktur kann mit anderen Worten dazu ausgeführt sein, um Fluide aus der Probenkammer zum Fluidauslass abzuführen. Dazu kann die Migrationsvorrichtung eine Fluidverbindung zwischen der Abführungsstruktur und dem Fluidauslass herstellen. Die Abführung von Fluiden mittels der Abführungsstruktur kann beispielsweise dadurch erreicht werden, dass die Abführungsstruktur fluidmechanisch mit einer Saugvorrichtung, z.B. einer Pumpe, verbunden ist.

Wenn nicht anders bezeichnet, betrifft eine im Kontext der vorliegenden Erfindung definierte Verbindung eine Fluidverbindung. Unter einer Fluidverbindung bzw. einer fluidmechanischen Verbindung zweier Elemente kann verstanden werden, dass eine Verbindung (z.B. in Form von Leitungen und/oder Kanälen) zwischen den beiden Elementen besteht, so dass eine Strömung eines Fluides von einem dieser Elemente zum anderen dieser Elemente ermöglicht wird.

Die Abführungsstruktur kann eine oder mehrere voneinander getrennte Einzelstrukturen, die jeweils zum Abführen eines Fluides nach außerhalb der Probenkammer sowie nach außerhalb der Migrationsmatrix zum Fluidauslass ausgeführt sind, umfassen. Beispielsweise kann die Abführungsstruktur eine erste Abführungsstruktur und eine zweite Abführungsstruktur aufweisen. Mit anderen Worten kann unter der Abführungsstruktur die Gesamtheit aller Abführungsstrukturen, die zum Abführen eines Fluides nach außerhalb der Probenkammer sowie nach außerhalb der Migrationsmatrix zum Fluidauslass ausgeführt sind, verstanden werden. Eine solche Abführungsstruktur kann beispielsweise zumindest teilweise in der Bodenfläche und/oder in der Seitenwand der Probenkammer ausgebildet sein, wobei die Abführungsstruktur auch nur in der Bodenfläche oder nur in der Seitenwand angeordnet sein kann. Das heißt dass die Abführungsstruktur eine in der Bodenfläche und/oder in der Seitenwand ausgebildete Unterstruktur, z.B. eine Abflussstruktur, aufweisen kann.

Zur Aufnahme mikroskopischer Stapelbilder können entweder die dafür benutzte Kamera oder die Migrationsvorrichtung auf einem x/y-Schlitten relativ zueinander oder auf einer Kreisbahn bewegt werden. Die Kamera kann dabei einen Streifen (Kreisbahn) mit vom Vergrößerungsfaktor bestimmter Breite überstreichen, was einen optischen Pfad festlegt. Um eine ungestörte optische Qualität der Aufnahmen zu bekommen, kann die Migrationsvorrichtung derart konstruiert sein, dass die Abführungsstruktur nicht durch diese optischen Pfade führt, d.h. dass die optischen Pfade frei von den geometrischen Konstruktionen der Abführstrukturen sind. Die Aufnahme der Stapelbilder erfolgt bevorzugt durch den Boden der Migrationsvorrichtung, der für die Messstrahlung durchlässige Bereiche aufweist.

Gemäß einem Ausführungsbeispiel der Erfindung weist die Migrationsvorrichtung ein Gehäuse auf, welches die Probenkammer enthält bzw. in welchem die Probenkammer ausgebildet ist. Weiterhin ist die Abführungsstruktur in dem Gehäuse ausgebildet und die Abführungsstruktur ist dazu ausgeführt, die Abführung eines Fluides nach außerhalb des Gehäuses zu ermöglichen.

Dabei kann das Gehäuse einteilig oder mehrteilig ausgebildet sein. Beispielsweise kann das Gehäuse ein Oberteil und ein Unterteil aufweisen, welche in einen montierten Zustand der Migrationsvorrichtung zusammenfügbar beziehungsweise zusammensetzbar sind. Das Gehäuse kann beispielsweise zumindest teilweise aus Kunststoff gefertigt sein, zum Beispiel mittels Spritzgussverfahren. Der Kunststoff kann zum Beispiel Eastman Tritan COPOLYESTER MX731 sein. Die Vorteile dieses Kunststoffs sind unter anderem: seine hohe Transparenz; die Verhinderung von Schlierenbildung; seine amorphe Eigenschaft; seine hohe Zähigkeit; seine gute chemische Verträglichkeit bei Kontakt mit Blut, In-Vitro-Chemikalien und Immersionsöl; seine Sterilisierbarkeit (gute Farbbeständigkeit bei Gamma- bzw. Ethylenoxid (ETO)-Sterilisation); bzw. seine FDA/ISO 10993 und USP Class VI Biokompatibilität. Der Kunststoff des Gehäuses kann beispielsweise so gewählt sein, dass im Zusammenspiel mit der Adaptation des Brechungsindex der Migrationsmatrix eine optische Transparenz in hoher mikroskopischer Qualität erreicht werden kann.

Das Gehäuse kann beispielsweise nur aus Spritzgussteilen und/oder aus käuflichen Standardkomponenten bestehen. Auf diese Weise kann die Migrationsvorrichtung sehr kostengünstig hergestellt werden.

Gemäß einer weiteren beispielhaften Ausführungsform der Erfindung weist die Probenkammer eine Bodenfläche bzw. einen Unterboden auf, wobei die Migrationsmatrix oder die Depotmatrix auf der Bodenfläche aufliegt und die Abführungsstruktur zumindest teilweise in der Bodenfläche ausgebildet ist. Das heißt, dass die Abführungsstruktur eine in der Bodenfläche ausgebildete mechanische Unterstruktur, z.B. eine Abflussstruktur (beispielsweise in Form einer Nut, eines verzweigten Nutsystems, einer Öffnung, eines Gitters, etc.), aufweisen kann.

Die Bodenfläche der Probenkammer kann beispielsweise durch eine Seitenwand der Probenkammer abgegrenzt sein. Weiterhin kann die Bodenfläche im Wesentlichen eben oder plan ausgebildet sein. Unter "im Wesentlichen" kann in diesem Zusammenhang verstanden werden, dass die ebene bzw. plane Bodenfläche Öffnungen, Vertiefungen und/oder Ausnehmungen (z.B. die im Kontext der Erfindung genannte Nut) aufweisen kann. Die Bodenfläche kann jedoch einen wesentlicher Teil, zum Beispiel mehr als 80 % der Bodenfläche, vorzugsweise mehr als 90 % der Bodenfläche, aufweisen, der bis auf fertigungstechnische Toleranzen eben bzw. plan ist. Die Bodenfläche kann alternativ auch gewölbt ausgebildet sein. Weiterhin kann die Bodenfläche auch einen gewölbten Bereich und einen planen Bereich aufweisen.

Eine solche in der Bodenfläche der Probenkammer ausgebildete Abführungsstruktur kann im Kontext der Erfindung auch als erste Abführungsstruktur bezeichnet sein. Beispielsweise kann die in der Bodenfläche ausgebildete Abführungsstruktur zumindest teilweise eine in der Bodenfläche angeordnete Nut umfassen. Die in der Bodenfläche ausgebildete Abführungsstruktur kann auch ein Abstandsgitter umfassen, das zwischen der Migrationsmatrix und der Bodenfläche angeordnet ist. Weiterhin kann die in der Bodenfläche ausgebildete Abführungsstruktur eine Bohrung oder mehrere Bohrungen, zum Beispiel durchgängige, kanalförmige Löcher, aufweisen, die in der Bodenfläche der Abführungsstruktur ausgebildet sind und in der Migrationsvorrichtung bzw. im Gehäuse der Migrationsvorrichtung in einen Kanal zusammengeführt werden.

Die Abführungsstruktur ist gemäß der Erfindung derart ausgebildet, dass die Matrix (Migrations- oder Depotmatrix) auf der Bodenfläche und zumindest teilweise auf der Abführungsstruktur aufliegt. Das heißt, dass die Abführungsstruktur unterhalb der Matrix der Migrationsvorrichtung angeordnet ist und/oder von dieser abgedeckt wird. Mittels einer solchen, in der Bodenfläche der Probenkammer angeordneten Abführungsstruktur können Gasblasen, die sich zwischen der Matrix und der Bodenfläche ausbilden, effektiv entfernt werden. Weiterhin können auf diese Weise auch Fluide durch die Migrationsmatrix aus der Probenkammer abgeführt werden. Auf diese Weise kann beispielsweise ein effektiver Trocknungsvorgang herbeigeführt werden, indem Luft aus der Probenkammer durch die Matrix mittels der in der Bodenfläche angeordneten Abführungsstruktur gesaugt wird, so dass auch die Matrix effektiv getrocknet wird.

Gemäß einer weiteren beispielhaften Ausführungsform der Erfindung weist die in der Bodenfläche ausgebildete Abführungsstruktur eine in der Bodenfläche angeordnete Nut auf.

Im Kontext der Erfindung kann die Nut auch als (nach oben geöffneter) Abführungskanal bezeichnet sein. Diese Nut kann beispielsweise in einen in der Migrationsvorrichtung ausgebildeten Kanal münden, der die Nut mit dem Fluidauslass verbindet. Dabei kann eine fluiddichte Verbindung zwischen der Nut und dem Fluidauslass bestehen. Die Nut kann beispielsweise von der Mitte der Bodenfläche der Probenkammer bis zum Rand der Bodenfläche der Probenkammer verlaufen. Beispielsweise kann die Nut eine Länge von ca. 4 mm bis 15 mm eine Breite von ca. 0,5 mm bis 1 mm und eine Tiefe von ca. 0,5 mm bis 1 mm aufweisen. Diese Maße der Nut können sich an den Maßen der Migrationsfläche (Boden) und/oder ihrem vom konstruktiven Aufbau definierten Wirkungsquerschnitt orientieren. Der Durchmesser der Migrationsfläche kann ca. 3 mm bis 10 mm, jener der Migrationsfläche samt Klemmbereich ca. 5 mm bis 20 mm betragen. Auf diese Weise kann eine Struktur bereitgestellt werden, welche das Bilden von Gasblasen verhindert und das Abführen von Fluiden ermöglicht.

Gemäß einer weiteren beispielhaften Ausführungsform der Erfindung weist die Abführungsstruktur einen in der Migrationsvorrichtung angeordneten, von der Probenkammer bis zum Fluidauslass durchgehenden ersten Fluidkanal auf.

Beispielsweise kann der Fluidkanal eine fluiddichte Verbindung zwischen dem Fluidauslass und einer Öffnung in einer Bodenfläche der Probenkammer und/oder in einer Seitenwand der Probenkammer ermöglichen.

Gemäß einer weiteren beispielhaften Ausführungsform der Erfindung umfasst die Probenkammer eine Seitenwand. Weiterhin umfasst die Abführungsstruktur eine in der Seitenwand der Probenkammer angeordnete Öffnung zum Absaugen von Fluiden aus der Probenkammer. Außerdem weist die Abführungsstruktur einen ringförmigen Fluidkanal auf, der in der Migrationsvorrichtung um die Probenkammer herum angeordnet ist. Die Abführungsstruktur weist eine Fluidverbindung zwischen dem ringförmigen Fluidkanal und dem Fluidauslass auf. Diese Fluidverbindung kann beispielsweise durch den ersten Fluidkanal bereitgestellt sein. Ferner weist die Abführungsstruktur eine Fluidverbindung zwischen der in der Seitenwand angeordneten Öffnung und dem ringförmigen Fluidkanal auf.

Mit anderen Worten kann die Abführungsstruktur einen Fluidkanal aufweisen, der die Probenkammer, beispielsweise ringförmig, umgibt. Dieser ringförmige Fluidkanal kann beispielsweise mit einer in der Bodenfläche der Probenkammer angeordneten Öffnung, zum Beispiel einer Nut, fluidmechanisch verbunden sein. Weiterhin kann der Fluidkanal auch mit der Öffnung bzw. mit Öffnungen, die in einer Seitenwand der Probenkammer ausgebildet ist bzw. sind, verbunden sein. Beispielsweise kann der Ringkanal mehrere Öffnungen, die sowohl in der Seitenwand als auch in der Bodenfläche der Probenkammer ausgebildet sein können, zu einem einzigen Fluidkanal, zum Beispiel dem vorher definierten ersten Fluidkanal, zusammenführen, so dass Fluide sowohl aus den Öffnungen in der Seitenwand als auch aus den Öffnungen in der Bodenfläche der Probenkammer zum Fluidauslass geleitet bzw. abgeführt werden können.

Diese Öffnung bzw. Öffnungen, die in der Seitenwand der Probenkammer ausgebildet sind, können auch als zweite Abführungsstruktur bezeichnet sein. Mittels dieser in der Seitenwand ausgebildeten Öffnung können Fluide von oberhalb der Migrationsmatrix abgeführt werden. Dieses Abführen kann beispielsweise für das Fixieren, Waschen, Färben und/oder Trocknen für die Bestimmung der Migrationsfähigkeit verwendet werden. Diese in der Seitenwand ausgebildeten Öffnungen können im Folgenden auch als Klemmring-Düsen bezeichnet sein.

Gemäß einer weiteren beispielhaften Ausführungsform der Erfindung weist die Probenkammer eine Bodenfläche und eine Seitenwand auf, wobei die Matrix (Migrations- oder Depotmatrix) auf der Bodenfläche aufliegt. Die Abführungsstruktur ist zumindest teilweise in der Bodenfläche ausgebildet, zum Beispiel als Nut. Weiterhin weist die Abführungsstruktur eine in der Seitenwand der Probenkammer angeordnete Öffnung auf. Dabei sind die in der Bodenfläche ausgebildete Abführungsstruktur und die in der Seitenwand angeordnete Öffnung mit demselben Fluidauslass verbunden. Die in der Bodenfläche ausgebildete Abführungsstruktur und die in der Seitenwand angeordnete Öffnung sind hinsichtlich ihrer Fluidverbindung entweder seriell (d.h. in Reihe) oder parallel geschaltet. Die Migrationsvorrichtung kann dabei eine einzige oder mehrere Öffnungen in der Seitenwand der Probenkammer aufweisen.

Mit anderen Worten können eine erste Abführungsstruktur und eine zweite Abführungsstruktur hinsichtlich ihrer Fluidverbindung mit dem Fluidauslass seriell oder parallel geschaltet bzw. angeordnet sein. Der Vorteil einer seriellen Schaltung der ersten Abführungsstruktur und der zweiten Abführungsstruktur untereinander liegt unter anderem darin, dass Fluide, die durch die in der seriellen Schaltung zuvorderst angeordnete Abführungsstruktur abgeführt werden, auch durch die in der seriellen Schaltung nachfolgende Abführungsstruktur geleitet werden, so dass die nachfolgende Abführungsstruktur gereinigt wird, wenn Fluide durch sie hindurch gespült werden.

Bei serieller Anordnung lässt sich der Differenzdruck zwischen Ober- und Unterseite der Matrix differenzierter, da strenger gekoppelt, mittels der Strömungsgeschwindigkeit einstellen, so dass ein reproduzierbarer Fluidtransport durch die Matrix erzeugt wird. Wasch-, Färbe- und Trocknungsprozesse können rascher und gleichmäßiger bei gleichzeitig verringertem Reagenzienverbrauch durchgeführt werden.

Bei einer seriellen Schaltung bzw. einer Reihenschaltung der in der Bodenfläche ausgebildeten Abführungsstruktur und der in der Seitenwand angeordneten Öffnung können die in der Bodenfläche ausgebildete Abführungsstruktur und die in der Seitenwand angeordneten Öffnungen hinsichtlich ihrer Fluidverbindung hintereinander angeordnet sein. Das heißt, dass der Fluidauslass beispielsweise direkt mit der in der Bodenfläche ausgebildeten Abführungsstruktur verbunden sein kann und die in der Bodenfläche ausgebildete Abführungsstruktur direkt mit der in der Seitenwand angeordneten Öffnung verbunden sein kann. Alternativ können der Fluidauslass direkt mit der in der Seitenwand angeordneten Öffnung verbunden und die in der Seitenwand angeordnete Öffnung direkt mit der in der Bodenfläche ausgebildeten Abführungsstruktur verbunden sein. Bei einer parallelen Schaltung der in der Bodenfläche ausgebildeten Abführungsstruktur und der in der Seitenwand angeordneten Öffnung mit dem Fluidauslass können die in der Bodenfläche ausgebildete Abführungsstruktur und die in der Seitenwand angeordnete Öffnung jeweils separat direkt mit dem Fluidauslass verbunden sein. Das schließt jedoch nicht aus, dass die in der Bodenfläche ausgebildete Abführungsstruktur und die in der Seitenwand angeordnete Öffnung separat mit demselben Fluidkanal (z.B. dem im Kontext der Erfindung genannten ersten Fluidkanal) verbunden sind, der den Fluidauslass mit den beiden Öffnungen verbindet.

Sowohl in der seriellen Schaltung als auch in der parallelen Schaltung der in der Bodenfläche ausgebildeten Abführungsstruktur und der in der Seitenwand angeordneten Öffnungen können die beiden Öffnungen mit demselben Fluidauslass verbunden sein. Dies hat den Vorteil, dass eine einzige Absaugpumpe bzw. Vakuumpumpe ausreicht, um mittels der in der Bodenfläche angeordneten Öffnung und der in der Seitenwand angeordneten Öffnung Fluide aus der Probenkammer abzuführen.

Gemäß einer weiteren beispielhaften Ausführungsform der Erfindung weist die Probenkammer eine Bodenfläche und eine Seitenwand auf. Weiterhin weist die Abführungsstruktur eine in der Bodenfläche ausgebildete Abführungsstruktur und eine in der Seitenwand angeordnete Öffnung auf. Ferner weist die Migrationsvorrichtung zumindest zwei separate bzw. voneinander getrennte Fluidauslässe auf, wobei die in der Bodenfläche ausgebildete Abführungsstruktur und die in der Seitenwand angeordnete Öffnung mit zwei separaten Fluidauslässen verbunden sind.

Mit anderen Worten können die in der Bodenfläche ausgebildete Abführungsstruktur mit einem ersten Fluidauslass und die in Seitenwand angeordnete Öffnung mit einem zweiten Fluidauslass verbunden sein.

Der Vorteil, der daraus resultiert, dass die in der Bodenfläche ausgebildete Abführungsstruktur und die in der Seitenwand angeordnete Öffnung mit separaten Fluidauslässen verbunden sind, liegt darin, dass die Abflüsse durch die in der Bodenfläche ausgebildete Abführungsstruktur und durch die in der Seitenwand angeordnete Öffnung separat kontrollierbar bzw. steuerbar sind.

Gemäß einer weiteren beispielhaften Ausführungsform der Erfindung weist die Migrationsvorrichtung ein Oberteil auf, welches eine von einer Seitenwand räumlich abgegrenzte Durchgangsöffnung und einen ersten Aufnahmebereich aufweist. Weiterhin weist die Migrationsvorrichtung ein Unterteil auf, welches einen zweiten Aufnahmebereich aufweist. Das Oberteil und das Unterteil sind in einen montierten Zustand der Migrationsvorrichtung zusammenfügbar. Im montierten Zustand der Migrationsvorrichtung ist die Migrationsmatrix im ersten Aufnahmebereich und im zweiten Aufnahmebereich angeordnet. Die Durchgangsöffnung bildet mit der Seitenwand die Probenkammer und das Unterteil bildet einen Boden der Probenkammer, wobei die Matrix auf dem Boden aufliegt. Weiterhin weist zumindest das Unterteil die Abführungsstruktur auf.

Gemäß einer weiteren beispielhaften Ausführungsform der Erfindung umfasst die Abführungsstruktur eine im Unterteil angeordnete Nut, wobei die Nut zumindest teilweise im zweiten Aufnahmebereich angeordnet ist, so dass die Nut im montierten Zustand der Migrationsvorrichtung von der Matrix (Migrations- oder Depotmatrix) abgedeckt ist.

Gemäß einer weiteren beispielhaften Ausführungsform der Erfindung sind das Oberteil und das Unterteil dazu ausgeführt, die Matrix in einem bestimmten Bereich der Matrix zwischen dem ersten Aufnahmebereich des Oberteils und des zweiten Aufnahmebereichs des Unterteils einzuklemmen.

Gemäß einer weiteren Ausführungsform der Erfindung ist der erste Aufnahmebereich als Klemmring ausgeführt, der eine Öffnung der Durchgangsöffnung umgibt. Weiterhin ist der erste Aufnahmebereich dazu ausgeführt, die Matrix, welche die Öffnung des Durchgangslochs abdeckt und den Boden der Probenkammer bildet, aufzunehmen.

Gemäß einer weiteren beispielhaften Ausführungsform der Erfindung weist das Oberteil einen ringförmigen Fluidkanal auf, welcher den Klemmring umgibt. Weiterhin weist das Oberteil einen Fluidauslass mit einem Anschlussstück zum Anschließen einer Vakuumpumpe an den Fluidauslass auf. Das Oberteil weist weiterhin einen weiteren Fluidkanal auf, der eine fluiddichte Verbindung zwischen dem Fluidauslass und dem ringförmigen Fluidkanal bereitstellt.

Mit anderen Worten kann das Gehäuse der Migrationsvorrichtung zweiteilig, aus dem Oberteil und dem Unterteil, aufgebaut sein. Der erste und der zweite Aufnahmebereich können jeweils einen Bereich des Oberteils bzw. des Unterteils bezeichnen, zwischen denen die Migrationsmatrix im montierten Zustand eingeklemmt ist. Der zweite Aufnahmebereich, der im Unterteil ausgebildet ist, kann eine ebene oder plane Fläche sein, die im montierten Zustand der Migrationsvorrichtung die Bodenfläche der Probenkammer ausbildet. Der erste Aufnahmebereich, der im Oberteil ausgebildet ist, kann ein die Probenkammer umgebender Klemmring sein, so dass die Migrationsmatrix und gegebenenfalls die Depotmatrix zwischen der Bodenfläche (das heißt dem zweiten Aufnahmebereich) und dem Klemmring (das heißt dem ersten Aufnahmebereich) eingeklemmt ist.

Der Vorteil eines solchen zweiteiligen Aufbaus liegt unter anderem darin, dass die Matrix auf einfache Weise zwischen dem Oberteil und dem Unterteil eingeklemmt werden kann.

Ein weiterer Aspekt der Erfindung betrifft die Verwendung einer Migrationsvorrichtung nach einem der vorangehenden Ansprüche zum Bestimmen der Migrationsfähigkeit amöboid beweglicher Zellen.

Ein weiterer Aspekt der Erfindung betrifft ein Verfahren zum Betreiben einer Migrationsvorrichtung, welches den Schritt des Bereitstellens einer Migrationsvorrichtung aufweisend eine Probenkammer, eine Migrationsmatrix und eine Abführungsstruktur zum Abführen eines Fluides aus der Migrationsvorrichtung zu einem Fluidauslass der Migrationsvorrichtung aufweist. Weiterhin weist das Verfahren den Schritt des Abführens eines Fluides nach außerhalb der Probenkammer und nach außerhalb der Migrationsmatrix durch die Abführungsstruktur zum Fluidauslass auf.

Das Verfahren kann im Weiteren das Bereitstellen einer Migrationsvorrichtung, wie sie im Kontext der Erfindung beschrieben ist, umfassen. Dabei kann die bereitgestellte Migrationsvorrichtung einzelne oder alle im Kontext der Erfindung beschriebenen Merkmale aufweisen.

Gemäß einer beispielhaften Ausführungsform der Erfindung ist ein Verfahren zum Entfernen einer Probe oder einer Behandlungsflüssigkeit beschrieben. Das Verfahren weist den Schritt des Einbringens der Probe oder der Behandlungsflüssigkeit in die Probenkammer auf. Der Schritt des Abführens eines Fluides umfasst ein Abführen zumindest eines Teils der Probe bzw. zumindest eines Teils der Behandlungsflüssigkeit durch die Abführungsstruktur.

Gemäß einer weiteren beispielhaften Ausführungsform der Erfindung ist ein Verfahren zum Auswaschen der Migrationsvorrichtung beschrieben, welches zusätzlich den Schritt des Einbringens einer Auswaschflüssigkeit in die Probenkammer aufweist. Der Schritt des Abführens eines Fluides umfasst ein Abführen zumindest eines Teils der Auswaschflüssigkeit durch die Abführungsstruktur.

Gemäß einer weiteren beispielhaften Ausführungsform der Erfindung ist ein Verfahren zum Trocknen der Migrationsvorrichtung beschrieben, welches zusätzlich den Schritt des Einbringens von Luft durch eine Eingangsöffnung der Migrationsvorrichtung in die Probenkammer durch Ansaugen durch die Abführungsstruktur aufweist. Der Schritt des Abführens eines Fluides zum Fluidauslass umfasst ein Abführen der angesaugten Luft durch die Abführungsstruktur.

Die Probenkammer kann eine Eingangsöffnung aufweisen, durch welches Fluide in die Probenkammer eingebracht werden können. Mit anderen Worten kann unter der Eingangsöffnung eine obere Öffnung der Probenkammer verstanden werden. Diese Eingangsöffnung kann beispielsweise verschließbar sein. Das heißt, dass die Migrationsvorrichtung Abdeckung zum Verschließen der Eingangsöffnung aufweisen kann.

Gemäß einer weiteren beispielhaften Ausführungsform der Erfindung ist ein Verfahren zum Vorbereiten einer Migrationsvorrichtung für eine optische Untersuchung der Migrationsmatrix beschrieben, welches zusätzlich die Schritte des Einbringens einer Flüssigkeit in die Matrix umfasst, wobei die eingebrachte Flüssigkeit, beispielsweise ein Immersionsöl, im Wesentlichen den gleichen Brechungsindex wie das Matrixmaterial aufweist, wodurch die Matrix transparent wird. Das Einbringen dieser Flüssigkeit kann über das Einführen einer Nadel durch eine Eingangsöffnung der Migrationsvorrichtung in die Migrationsmatrix erfolgen, wobei die Nadel durch die Matrix hindurch und zumindest teilweise in die Abführungsstruktur eindringt.

Gemäß einer weiteren beispielhaften Ausführungsform der Erfindung ist ein Verfahren zur Migrationsanalyse beschrieben, welches die Schritte des Einbringens einer Probenflüssigkeit in die Probenkammer, des Fixierens von Zellen der Probenflüssigkeit durch Einbringen einer Fixierflüssigkeit, des Färbens der Zellen durch Einbringen einer Färbeflüssigkeit in die Probenkammer, und des optischen Vermessens einer Migrationsfähigkeit der Zellen aufweist, wobei sämtliche Schritte dieses Verfahrens mit der Migrationsvorrichtung durchgeführt werden. Dabei kann verstanden werden, dass sämtliche Schritte des Verfahrens innerhalb oder mittels der Migrationsvorrichtung durchgeführt werden.

Die im Kontext der Erfindung beschriebenen Verfahrensschritte können, wenn nicht anders bezeichnet, sowohl in der beschriebenen Reihenfolge als auch in anderen Reihenfolgen durchgeführt werden. Weiterhin können die erfindungsgemäßen Verfahren bzw. deren Schritte mit der im Kontext der Erfindung beschriebenen Migrationsvorrichtung durchgeführt werden. Die beschriebenen Ausführungsformen betreffen gleichermaßen eine Migrationsvorrichtung, die Verwendung einer Migrationsvorrichtung und Verfahren zum Betreiben der Migrationsvorrichtung. Synergetische Effekte können sich aus verschiedenen Kombinationen der Ausführungsformen ergeben, auch wenn diese im Folgenden nicht explizit beschrieben sind. Merkmale, welche die Vorrichtung charakterisieren, können auch das Verfahren zum Betreiben der Vorrichtung charakterisieren. Umgekehrt können Merkmale, die definieren, wie die Vorrichtung zu betreiben ist, selbst auch ein Merkmal der Vorrichtung sein.

Weitere Merkmale, Vorteile und Anwendungsmöglichkeiten der Erfindung ergeben sich aus der nachfolgenden Beschreibung der Ausführungsbeispiele und Figuren. Dabei bilden alle beschriebenen und/oder bildlich dargestellten Merkmale für sich und in beliebiger Kombination den Gegenstand der Erfindung auch unabhängig von ihrer Zusammensetzung in den einzelnen Ansprüche oder deren Rückbezügen.

### Kurze Beschreibung der Figuren:

- Fig. 1: zeigt eine Draufsicht einer Migrationsvorrichtung gemäß einem Ausführungsbeispiel der Erfindung;
- Fig. 2: zeigt eine Seitenansicht einer Migrationsvorrichtung gemäß einem weiteren Ausführungsbeispiel der Erfindung;
- Fig. 3: zeigt eine Seitenansicht einer Migrationsvorrichtung gemäß einem weiteren Ausführungsbeispiel der Erfindung;
- Fig. 4: zeigt eine Seitenansicht einer Migrationsvorrichtung gemäß einem weiteren Ausführungsbeispiel der Erfindung;
- Fig. 5A, Fig. 5B, Fig. 5C und Fig. 5D: zeigen verschiedene Ansichten einer Migrationsvorrichtung gemäß einem weiteren Ausführungsbeispiel der Erfindung;
- Fig. 5E und Fig. 5F: zeigen jeweils eine Migrationsvorrichtung gemäß einem Ausführungsbeispiel der Erfindung beim Betreiben der Migrationsvorrichtung;
- Fig. 6: zeigt eine Migrationsvorrichtung mit zwei Abführungsstrukturen, die mit unterschiedlichen Fluidauslässen verbunden sind, gemäß einem weiteren Ausführungsbeispiel der Erfindung;
- Fig. 7: zeigt eine Migrationsvorrichtung mit zwei Abführungsstrukturen, die mit demselben Fluidauslass verbunden sind und die parallel geschaltet sind, gemäß einem weiteren Ausführungsbeispiel der Erfindung;
- Fig. 8: zeigt eine Migrationsvorrichtung mit zwei Abführungsstrukturen, die mit demselben Fluidauslass verbunden sind und die in Reihe geschaltet sind, gemäß einem weiteren Ausführungsbeispiel der Erfindung;
- Fig. 9: zeigt eine Migrationsvorrichtung gemäß einem weiteren Ausführungsbeispiel der Erfindung;
- Fig. 10: zeigt ein Flussdiagramm für ein Verfahren zum Betreiben einer Migrationsvorrichtung gemäß einem Ausführungsbeispiel der Erfindung;
- Fig. 11: zeigt mehrere Flussdiagramme für Verfahren gemäß Ausführungsbeispielen der Erfindung;
- Fig. 12: zeigt ein Flussdiagramm für ein Verfahren für die Migrationsanalyse gemäß einem Ausführungsbeispiel der Erfindung.

Die Figuren sind schematisch und nicht zwingendermaßen maßstabsgetreu dargestellt. Sollten in unterschiedlichen Figuren Elemente mit gleichen Bezugszeichen bezeichnet sein, so bezeichnen diese gleiche, ähnliche oder einander entsprechende Elemente. Gleiche, ähnliche oder einander entsprechende Elemente können in verschiedenen Figuren aber auch unterschiedlich bezeichnet sein.

### Detaillierte Beschreibung von Ausführungsbeispielen:

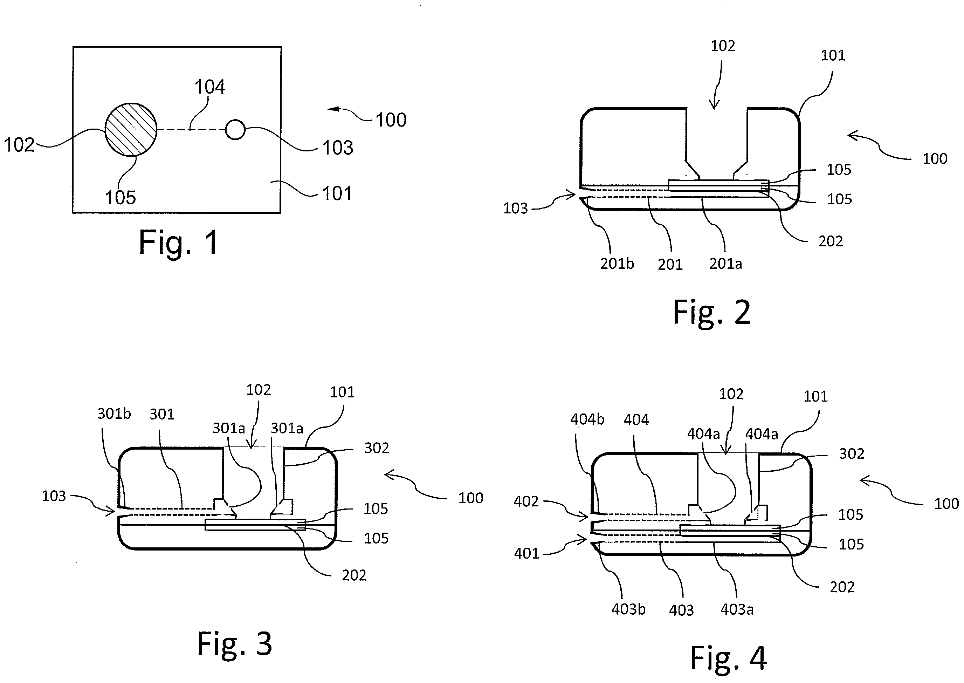

In Fig. 1 ist eine Migrationsvorrichtung 100 vereinfacht dargestellt, welche ein Gehäuse 101 aufweist. Die Migrationsvorrichtung 100 enthält weiterhin eine Proben- bzw. Migrationskammer 102, die im Gehäuse 101 der Migrationsvorrichtung 100 ausgebildet ist. Gemäß diesem Ausführungsbeispiel ist die Probenkammer 102 als kreisförmige Vertiefung bzw. Ausnehmung im Gehäuse 101 ausgebildet. Die Probenkammer 102 kann jedoch auch eine andere Querschnittsform aufweisen. Weiterhin enthält die Migrationsvorrichtung 100 eine Migrationsmatrix 105, die in der Probenkammer 102 angeordnet ist. Außerdem weist die Migrationsvorrichtung 100 einen Fluidauslass 103 auf. Die Migrationsvorrichtung 100 weist ferner eine Abführungsstruktur 104 auf, die zum Abführen eines Fluides nach außerhalb der Probenkammer 102 sowie nach außerhalb der Migrationsmatrix 105 zum Fluidauslass 103 ausgeführt ist. Mit anderen Worten stellt die Abführungsstruktur 104 eine Fluidverbindung zwischen der Probenkammer 102 und dem Fluidauslass 103 her, so dass Fluide aus der Probenkammer 102 zum Fluidauslass 103 abgeführt werden können.

In Fig. 2 ist eine Migrationsvorrichtung 100 gemäß einem weiteren Ausführungsbeispiel der Erfindung dargestellt, welche ein Gehäuse 101 mit einer Probenkammer 102 und einem Fluidauslass 103 enthält. In diesem Ausführungsbeispiel ist die obere Öffnung der Probenkammer 102 die im Kontext der Erfindung beschriebene Eingangsöffnung der Probenkammer 102. Am Boden 202 der Probenkammer ist die Migrationsmatrix 105 angeordnet, so dass diese auf dem Boden 202 der Probenkammer aufliegt. Weiterhin ist die Abführungsstruktur 201 zumindest teilweise im Boden 202 der Probenkammer 102 ausgebildet, so dass die im Boden 202 ausgebildete Abführungsstruktur zumindest teilweise von der Migrationsmatrix 105 abgedeckt wird. Beispielsweise umfasst die Abführungsstruktur 201 eine im Boden ausgebildete Abflussstruktur 201a, z.B. eine im Boden ausgebildete Öffnung, Nut oder Nutsystem. Die Abführungsstruktur 201 umfasst weiterhin eine Auslassöffnung 201b. Weiterhin weist die Abführungsstruktur 201 einen ersten Fluidkanal auf, der von der im Boden ausgebildeten Abflussstruktur 201a zur Auslassöffnung 201b bzw. dem Fluidauslass 103 führt. Auf diese Weise werden Fluide, die aus der Probenkammer 102 durch die Abführungsstruktur 201 zum Fluidauslass 103 abgeführt werden, durch die Migrationsmatrix 105 geleitet. Die in Fig. 2 beispielhaft dargestellte Abführungsstruktur 201 kann im Kontext der Erfindung auch als erste Abführungsstruktur bezeichnet sein.

In Fig. 3 ist eine Migrationsvorrichtung 100 gemäß einem weiteren Ausführungsbeispiel der Erfindung dargestellt. Die Migrationsvorrichtung 100 weist ein Gehäuse 101 mit einer Probenkammer 102, eine Migrationsmatrix 105 und einem Fluidauslass 103 auf. Die Abführungsstruktur 301 der Migrationsvorrichtung 100 gemäß Fig. 3 ist derart ausgebildet, dass sie von der Seitenwand 302 der Probenkammer zum Fluidauslass 103 führt. Das heißt, dass die Abführungsstruktur 301 in die Seitenwand 302 der Probenkammer 102 mündet. Mit anderen Worten weist die Abführungsstruktur 301 eine oder mehrere in der Seitenwand der Probenkammer 102 ausgebildete Öffnungen 301a auf. Weiterhin weist die Abführungsstruktur 301 einen Fluidkanal auf, der von den in der Seitenwand ausgebildeten Öffnungen 301a zur Auslassöffnung 301b führt. Auf diese Weise müssen Fluide, die aus der Probenkammer 102 zum Fluidauslass 103 abgeführt werden, nicht durch die Migrationsmatrix 105 geleitet werden. Die in Fig. 3 beispielhaft dargestellte Abführungsstruktur 301 kann im Kontext der Erfindung auch als zweite Abführungsstruktur bezeichnet sein.

In Fig. 4 ist eine Migrationsvorrichtung 100 gemäß einem weiteren Ausführungsbeispiel der Erfindung dargestellt. Die Migrationsvorrichtung 100 weist ein Gehäuse 101 mit einer Probenkammer 102, einer Migrationsmatrix 105, einem ersten Fluidauslass 401 und einem zweiten Fluidauslass 402 auf. Die Abführungsstruktur umfasst eine erste Abführungsstruktur 403 und eine zweite Abführungsstruktur 404 auf. Die erste Abführungsstruktur 403 ist derart ausgebildet, dass Fluide von der Bodenfläche 202 der Probenkammer 102 durch die erste Abführungsstruktur 403 zum ersten Fluidauslass 401 abgeführt werden. Die erste Abführungsstruktur 403 umfasst eine im Boden ausgebildete Abflussstruktur 403a, einen ersten Fluidkanal und eine erste Auslassöffnung 403b. Auf diese Weise werden Fluide, die durch die erste Abführungsstruktur 403 abgeführt werden, durch die Migrationsmatrix 105 geleitet. Die zweite Abführungsstruktur 404 ist derart ausgebildet, dass Fluide aus der Probenkammer 102 von der Seitenwand 302 durch die zweite Abführungsstruktur 404 zum zweiten Fluidauslass 402 abgeführt werden. Die zweite Abführungsstruktur 404 umfasst eine oder mehrere in der Seitenwand ausgebildete Öffnungen 404a, einen zweiten Fluidkanal und eine zweite Auslassöffnung 404b. In diesem Ausführungsbeispiel ist dargestellt, dass die erste Abführungsstruktur 403 und die zweite Abführungsstruktur 404 mit separaten Fluidauslässen 401 und 402 verbunden sind. Jedoch können die erste Abführungsstruktur 403 und die zweite Abführungsstruktur 404 auch mit demselben Fluidauslass verbunden und in Reihe oder parallel geschaltet sein.

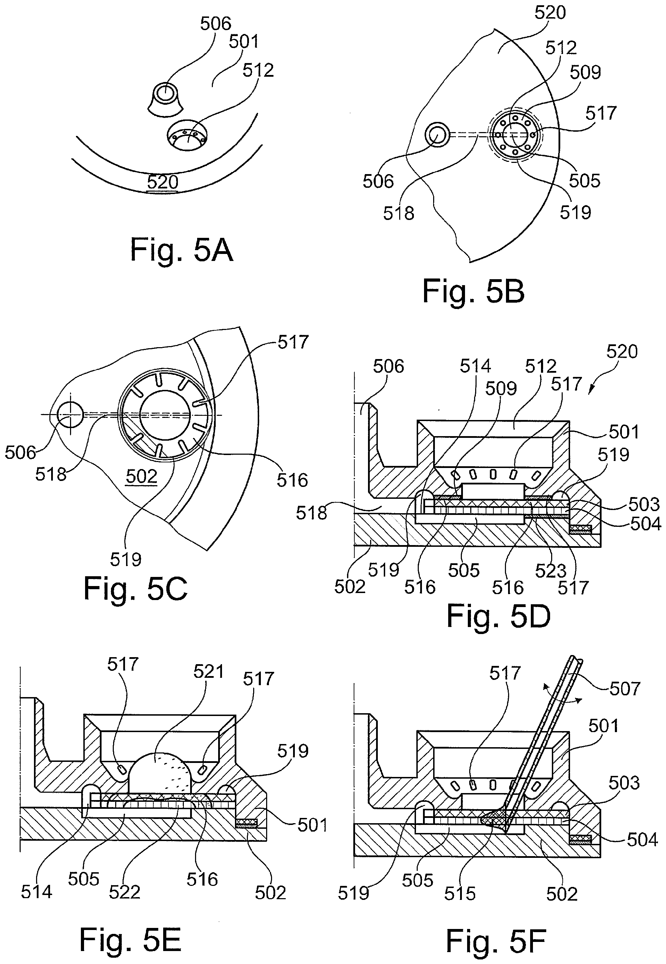

In den Fig. 5A, Fig. 5B, Fig. 5C und Fig. 5D ist eine Migrationsvorrichtung gemäß einem weiteren Ausführungsbeispiel der Erfindung in verschiedenen Ansichten dargestellt. Das Gehäuse der Migrationsvorrichtung weist dabei ein Oberteil 501 und ein Unterteil 502 auf. In Fig. 5A ist das Oberteil 501 in einer 3D-Ansicht gezeigt. Fig. 5B zeigt das Oberteil und das Unterteil in einer Draufsicht. Fig. 5C zeigt das Unterteil sowie die darauf angeordnete Matrix nach dem Stanzvorgang. Fig. 5D zeigt eine Schnittdarstellung der Migrationsvorrichtung 520.

Wie in Fig. 5D dargestellt, weist die Migrationsvorrichtung 520 ein Oberteil 501 und ein Unterteil 502 auf, zwischen denen die Matrix 503, 504 angeordnet bzw. eingeklemmt ist. In diesem Ausführungsbeispiel umfasst die Matrix zwei schichtartig übereinander angeordnete Matrices 503 und 504, wobei die obere Matrix eine Migrationsmatrix 503 und die untere Matrix eine Depotmatrix 504 ist. Das Oberteil 501 weist weiterhin den Fluidauslass 506 auf, der einen zentralen Absauganschluss bzw. Absaugnippel, zum Beispiel ein Luer, aufweist. Weiterhin enthält das Oberteil 501 eine Durchgangsöffnung, welche die Probenkammer 512 ausbildet. Die obere Öffnung der Probenkammer 512 bildet in diesem Ausführungsbeispiel die Eingangsöffnung der Probenkammer 512. Das Oberteil 501 weist weiterhin einen ersten Aufnahmebereich auf, der als Klemmring 509 bzw. ringförmiger Klemmbereich ausgebildet ist. Das Unterteil 502 umfasst einen zweiten Aufnahmebereich 523, so dass die Matrices 503, 504 in einem Klemmbereich 516 zwischen dem ersten Aufnahmebereich 509 (das heißt dem Klemmring) und dem zweiten Aufnahmebereich 523 eingeklemmt wird. Der Klemmbereich 516 bildet eine Saugstrecke, die nachfolgend beschrieben ist. Da das Unterteil 502, wie im Folgenden erläutert, eine Nut 505 bzw. ein Nutsystem aufweist, kann der zweite Aufnahmebereich 523 durch die Nut 505 unterbrochen sein.

Die Migrationsvorrichtung 520 weist weiterhin eine Abführungsstruktur auf, die mehrere einzelne Unterstrukturen umfasst. Unter anderem weist die Abführungsstruktur eine in der Bodenfläche des Unterteils 502 ausgebildete Nut 505, einen im Oberteil ausgebildeten ringförmigen, die Probenkammer 512 umgebenden ringförmigen Fluidkanal 519, einen ersten Fluidkanal 518 und mehrere in der Seitenwand der Probenkammer 512 angeordnete Öffnungen 517, die fluidmechanisch gesehen mit dem ringförmigen Fluidkanal 519 verbunden sind, auf. Im Folgenden kann die Nut 505 auch als Absaug- bzw. Abführungskanal bezeichnet sein. Der ringförmige Fluidkanal 519 kann auch als Ringkanal und die in der Seitenwand angeordneten Öffnungen 517 können auch als Klemmring-Düsen bezeichnet sein. Der ringförmige Fluidkanal 519 und die Nut 505 sind an der Verbindungsstelle 514 miteinander verbunden. Die in der Bodenfläche des Unterteils 502 ausgebildete Nut 505 ist fluidtechnisch mit dem Ringkanal 519 verbunden, der wiederum fluidtechnisch mit dem ersten Fluidkanal 518 verbunden ist, der wiederum fluidtechnisch mit dem Fluidauslass 506 verbunden ist.

Die Migrationsvorrichtung umfasst eine Migrationsmatrix 503 mit einer für die Migration geeigneten Porenweite und eine Depotmatrix 504 zur Aufnahme eines Wirkstoffs. Die beiden Matrices 503, 504 werden vom Klemmbereich bzw. Klemmring 509 des Oberteiles 501 mit einer derart bemessenen Presskraft an den Aufnahmebereich 523 des Unterteils 502 gedrückt, dass eine ausreichende Dichtheit über den gesamten Umfang des Klemmbereichs erreicht wird, aber gleichzeitig noch genügend Porosität in den Matrix-Abschnitten 503, 504 unterhalb des Klemmringes 509 bestehen bleibt, um eine kapillare Kriechstrecke bzw. Saugstrecke für die Probe im Klemmbereich 516 zwischen Probenkammer 512 und Ringkanal 519 zu definieren.

Die Abführungsstruktur weist eine unterhalb der Matrices im Boden angeordnete erste Abführungsstruktur auf, z.B. einen schmalen Absaugkanal bzw. eine Nut 505, oder ein verzweigtes Nutsystem, die mit dem Ringkanal 519 sowie mit dem ersten Fluidkanal 518 verbunden ist, um einen Fluidtransport (z.B. von der Probenflüssigkeit, Luft, Reagenzien, Immersionsöl etc.) durch die beiden Matrices 503, 504 zu ermöglichen und somit einen raschen und effektiven Materie-Austausch zu erlauben bzw. einen Luftstau und Luftblasenbildung (siehe 522) bei Erstbenetzung zu verhindern. Bei Anschluss einer Luftpumpe (z.B. Absaugpumpe, Vakuumpumpe) an den Absaugnippel des Fluidauslasses 506 wird bei ausreichendem Unterdruck der Hauptanteil der z.B. mit einer Reagenz oder Waschlösung gefüllten Probenkammer 512 über die Klemmring-Düsen 517, Ringkanal 519 und ersten Fluidkanal 518 entleert.

Fig. 5E zeigt die Erstbenetzung der Migrationsmatrix in der in den Fig. 5A bis Fig. 5D gezeigten Migrationsvorrichtung gemäß dem Ausführungsbeispiel der Erfindung. Die Probe, z.B. Vollblut, wird vor der Aufbringung auf die Migrationsmatrix 503 mit einem Puffer verdünnt. Ein Teil des wässrigen Anteils der Probe durchdringt rasch Migrations- und Depotmatrix 503, 504 und löst in der Depotmatrix 504 den Wirkstoff. Dadurch entsteht ein Wirkstoffgradient für die zu analysierenden Zellen in der Probe. Durch geeignete Wahl eines Trägermaterials kann die Freisetzungskinetik des Wirkstoffs kontrolliert werden, um so den Gradienten für eine bestimmte Zeit aufrecht zu erhalten.

Es hat sich als vorteilhaft erwiesen, eine Vorbenetzung der Matrices 503, 504 mit einem Puffer durchzuführen. Danach wird die verdünnte Probe auf die Migrationsmatrix 503 aufgebracht. Nach einer vorbestimmten Inkubationszeit (typischerweise 15 bis 30 Minuten) wird der Migrationsprozess durch Fixierung der Zellen gestoppt, z.B. durch Formaldehyd. Danach erfolgt ein ein- oder mehrstufiger Prozess, in dem die Zellen bzw. Zellkomponenten gefärbt werden. Um optische Transparenz zu erreichen, wird die Luft aus den Poren der Matrices durch Verwendung einer Flüssigkeit 515 (z.B. Immersionsöl) mit einem entsprechenden Brechungsindex verdrängt und über eine optische Analyse (z.B. mikroskopische Aufnahmen) eine Ermittlung der Migrationsstrecken der Zellen vorgenommen. Die optische Analyse erfolgt bevorzugt durch den zumindest in Bereichen optisch durchlässigen bzw. transparenten Boden der Vorrichtung.

Die Unterhalb der Depotmatrix im Unterteil 502 ausgebildete erste Abführungsstruktur verschafft eine Möglichkeit, um Gase und Luftblasen rasch entweichen zu lassen, z.B. in Form eines Absaug-Kanales (z.B. Nut 505, Nutsystem oder Gitterstruktur), sowie ein rasches und effizientes Trocknen der Matrices 503, 504.

Um einen reproduzierbaren Prozess sicherzustellen und ein Austrocknen zu vermeiden, kann während der Inkubation eine konstante Temperatur, z.B. 37°C, und eine hohe Luftfeuchtigkeit erforderlich sein. Vorteilhafterweise wird dies dadurch erzielt, dass die Probenkammern 512 der Migrationsvorrichtung 520 relativ klein ausgeführt sind und während der Inkubationszeit verschlossen sowie von oben und bodenseitig temperiert werden.

Fixier- und Färbeprozesse werden durch einen Abfluss unterhalb der Matrices, z.B. in Form der Nut 505, nur durch Anlegen einer ausreichenden Strömung und des sich daraus einstellenden Unterdruckes, ohne zusätzliche Bohrung, Kanäle, Ventile etc., erleichtert, beschleunigt und qualitativ verbessert, so dass bei sich wiederholenden Teilschritten signifikant Zeit eingespart werden kann, da der Fluidaustausch (Reagenzien) sowie der Trocknungsvorgang (Luft) nicht ausschließlich durch Diffusion, sondern zeitsparender und vollständiger durch Konvektion erfolgen kann.

Weiterhin haben sich tröpfchenweise dosierte Zwischen-Waschprozesse als vorteilhaft erwiesen, da sich durch das alternierende Saugen von Luft und Wasser unter Unterdruck die Effizienz des Waschens steigern lässt.

Fig. 5F zeigt das Einbringen von einer Flüssigkeit, beispielsweise eines Immersionsöles, in die Migrationsmatrix. Das Einbringen des Immersionsöls 515, kann mit dem Risiko verbunden sein, dass das auf die Migrationsmatrix 503 aufgetragene Immersionsöl 515 wegen seiner erhöhten Viskosität und der, bedingt durch die vorgelagerten Fluidprozesse oder auch produktionsbedingt, lokal unterschiedlich benetzbaren Teiloberflächen zeitlich verzögert in die Matrix eindringt und die von der Fluidfront verdrängte Luft aus den Matrices 503, 504 nicht nach oben bzw. nach außen entweichen kann und sich auf diese Weise unter der Depotmatrix 504 bzw. zwischen den beiden Matrices 503, 504 mehr oder weniger große Luftblasen sammeln. Dieser Effekt kann sich verstärken, wenn die Porenweite der Depotmatrix 504 kleiner ist als die der Migrationsmatrix 503. Üblicherweise ist die Porenweite der Depotmatrix geringer, um eine möglichst große Oberfläche für die Abgabe des Lockstoffs zur Verfügung zu haben. Vorteilhaft ist deshalb die Einbringung des Immersionsöles 515 mit einer schräg zulaufenden Nadel 507, z.B. einer Spritzennadel, indem der schräg zulaufende Teil der Nadelspitze beide Matrices im Bereich der Nut 505 des Unterbodens durchdringt und das Reagenz seitlich in und unter beide Matrices gleichzeitig eindringen kann. Dieser Prozess kann durch Drehen der Nadel während der Einbringung und/oder gleichzeitiges Anlegen von Unterdruck an der Nut 505 noch verbessert werden.

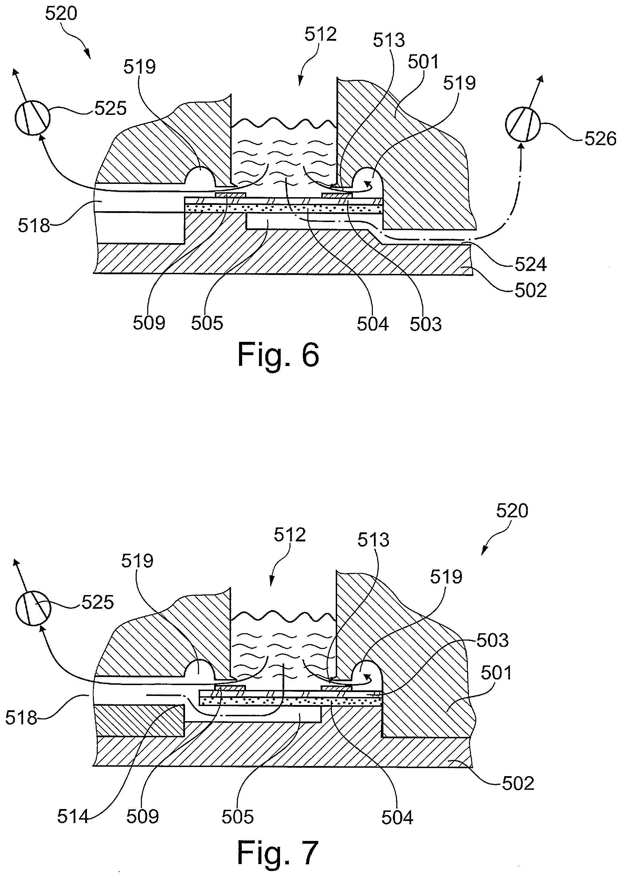

Fig. 6 zeigt eine Migrationsvorrichtung 520 gemäß einem weiteren Ausführungsbeispiel der Erfindung. Die Migrationsvorrichtung weist ein Oberteil 501 und ein Unterteil 502 auf. Im Oberteil ist die Probenkammer 512 ausgebildet, an deren Boden die Migrationsmatrix 503 und die Depotmatrix 504 angeordnet sind, welche zwischen dem Klemmring 509 des Oberteils 501 und dem Unterteil 502 eingeklemmt sind. Insgesamt weist die Migrationsvorrichtung eine Abführungsstruktur auf, die eine im Unterteil ausgebildete Nut 505 und in der Seitenwand der Probenkammer ausgebildete Öffnungen 513 enthält. Die in der Seitenwand ausgebildeten Öffnungen 513 sind über den Ringkanal 519 mit dem ersten Fluidkanal 518 und somit mit einer ersten Absaugpumpe bzw. Vakuumpumpe 525 verbunden. Die im Unterteil ausgebildete Nut 505 ist über einen zweiten Fluidkanal 524 mit einem zweiten Fluidauslass und einer zweiten Vakuumpumpe 526 verbunden. Das heißt, dass die unterhalb der Migrationsmatrix 503 und Depotmatrix 504 angeordnete Nut 505 mit einem anderen Fluidauslass als die in der Seitenwand ausgebildeten Öffnungen 513 verbunden sind, so dass die Flüsse durch die Nut 505 und durch die in der Seitenwand ausgebildeten Öffnungen 513 separat gesteuert werden können.

In Fig. 7 ist eine Migrationsvorrichtung 520 gemäß einem weiteren Ausführungsbeispiel der Erfindung dargestellt, welches ebenfalls ein Oberteil 501 und ein Unterteil 502 aufweist. Im Oberteil 501 ist die Probenkammer 512 ausgebildet. Zwischen dem Klemmring 509 des Oberteils und dem Unterteil 502, welches den Boden der Probenkammer bildet, sind die Migrationsmatrix 503 und die Depotmatrix 504 eingeklemmt. Die Abführungsstruktur weist eine im Unterteil ausgebildete Nut 505, in der Seitenwand der Probenkammer 512 ausgebildete Öffnungen 513, einen Ringkanal 519 sowie einen Fluidkanal 518 auf. Dabei sind die in der Seitenwand angeordneten Öffnungen 513 und die im Unterteil 502 ausgebildete Nut 505 mit demselben Fluidkanal 518 und somit mit derselben Absaugpumpe bzw. Vakuumpumpe 525 verbunden. Die Nut 505 ist dabei über die Verbindungsstelle 514 mit dem Fluidkanal 518 verbunden. Gemäß diesem Ausführungsbeispiel sind die Nut 505 und die in der Seitenwand ausgebildeten Öffnungen 513 fluidtechnisch gesehen parallel geschaltet. Das heißt, dass die Nut 505 und die in der Seitenwand ausgebildeten Öffnungen 513 separat am Fluidkanal 518 angeschlossen sind.

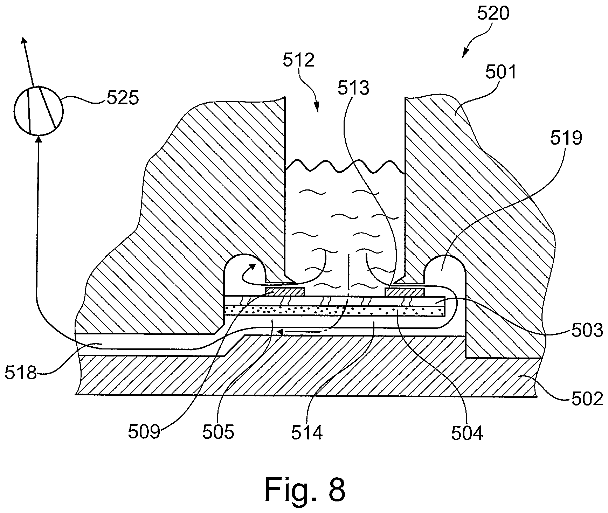

In Fig. 8 ist eine Migrationsvorrichtung 520 dargestellt, die ein Oberteil 501 und ein Unterteil 502 aufweist, wobei das Oberteil die Probenkammer 512 enthält. Die Migrationsmatrix 503 und die Depotmatrix 504 sind zwischen dem Klemmring 509 des Oberteils 501 und dem Unterteil 502 eingeklemmt. Die Abführungsstruktur weist eine im Unterteil ausgebildete Nut 505, einen Ringkanal 519, in der Seitenwand ausgebildete Öffnungen 513 sowie einen Fluidkanal 518 auf, die hinsichtlich der Fluidverbindung in Reihe bzw. seriell geschaltet sind und mit der Absaugpumpe bzw. Vakuumpumpe 525 verbunden sind. Der Fluidkanal 518 ist mit der Nut 505, die Nut 505 mit dem Ringkanal 519 und der Ringkanal über die in der Seitenwand ausgebildeten Öffnungen 513 mit der Probenkammer 512 fluidmechanisch verbunden. Das heißt, dass Fluide, die durch die in der Seitenwand ausgebildeten Öffnungen 513 abgeführt werden, durch die Nut 505 geleitet werden. Dies wiederum hat den Vorteil, dass die Nut 505 durch die Flüssigkeit, die beim Absaugen durch die Öffnungen 513 der Seitenwand durch die Nut gespült 505 wird, ausgewaschen wird.

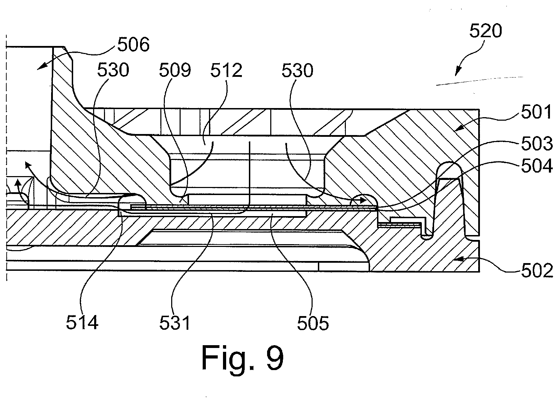

In Fig. 9 ist eine Migrationsvorrichtung 520 gemäß einem weiteren Ausführungsbeispiel der Erfindung dargestellt. Es ist gezeigt, dass im Unterteil 502 eine Nut ausgebildet ist, die eine erste Abführungsstruktur bildet. Im Oberteil sind in der Seitenwand der Probenkammer 512 Öffnungen enthalten (nicht explizit dargestellt), die eine zweite Abführungsstruktur bilden. Die Nut 505 und die Öffnungen der Seitenwand sind fluidmechanisch mit dem Fluidkanal verbunden, der den im Kontext der Erfindung definierten ersten Fluidkanal darstellt. Die Nut 505 ist über die Verbindungsstelle 514 mit dem Fluidkanal verbunden. Außerdem sind Pfeile 530 und 531 gezeigt, welche die Strömung des Fluides bei dessen Absaugung darstellen. Der Pfeil 530 zeigt die Strömung des Fluides, das durch die in der Seitenwand ausgebildeten Öffnungen abgesaugt wird und der Pfeil 531 zeigt die Strömung des Fluides, das durch die Nut 505 abgesaugt wird.

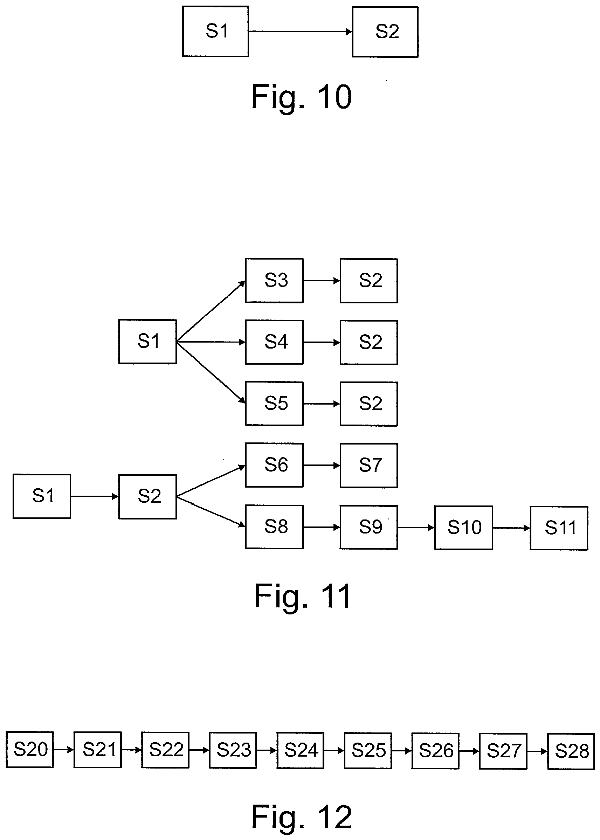

In Fig. 10 ist ein Flussdiagramm für ein Verfahren zum Betreiben einer Migrationsvorrichtung gemäß einem Ausführungsbeispiel der Erfindung dargestellt. Das Verfahren beinhaltet den Schritt S1 des Bereitstellens einer Migrationsvorrichtung aufweisend eine Probenkammer, eine Migrationsmatrix und eine Abführungsstruktur zum Abführen eines Fluides aus der Migrationsvorrichtung zu einem Fluidauslass der Migrationsvorrichtung. Weiterhin weist das Verfahren den Schritt S2 des Abführens eines Fluides aus der Probenkammer und/oder aus der Migrationsmatrix durch die Abführungsstruktur zum Fluidauslass auf.

In Fig. 11 sind verschiedene Flussdiagramme für verschiedene Verfahren gemäß Ausführungsbeispielen der Erfindung dargestellt.

Ein Ausführungsbeispiel der Erfindung betrifft ein Verfahren zum Entfernen einer Probe oder einer Behandlungsflüssigkeit, welches den Schritt S1 des Bereitstellens der Migrationsvorrichtung, den Schritt S3 des Einbringens der Probe oder der Behandlungsflüssigkeit in die Probenkammer und den Schritt S2 des Abführens eines Fluides aus der Probenkammer und/oder aus der Migrationsmatrix durch die Abführungsstruktur zum Fluidauslass zum Abführen zumindest eines Teils der Probe bzw. zumindest eines Teils der Behandlungsflüssigkeit durch die Abführungsstruktur umfasst.

Ein weiteres Ausführungsbeispiel der Erfindung betrifft ein Verfahren zum Auswaschen der Migrationsvorrichtung, welches den Schritt des Bereitstellens der Migrationsvorrichtung S1, den Schritt S4 des Einbringens einer Auswaschflüssigkeit in die Probenkammer und den Schritt S2 des Abführens zumindest eines Teils der Auswaschflüssigkeit durch die Abführungsstruktur umfasst.

Ein weiteres Ausführungsbeispiel der Erfindung betrifft ein Verfahren zum Trocknen der Migrationsvorrichtung, welches den Schritt S1 des Bereitstellens der Migrationsvorrichtung, den Schritt S5 des Einbringens von Luft durch eine Durchgangsöffnung der Migrationsvorrichtung in die Probenkammer durch Ansaugen durch die Abführungsstruktur, und den Schritt S2 des Abführens der angesaugten Luft durch die Abführungsstruktur umfasst.

Ein weiteres Ausführungsbeispiel der Erfindung betrifft ein Verfahren zum Vorbereiten einer Migrationsvorrichtung für eine optische Untersuchung der Migrationsmatrix, welches die Schritte S1 und S2, die oben näher beschrieben sind, beinhaltet. Außerdem umfasst das Verfahren den Schritt S6 des Einbringens einer Flüssigkeit mit einem Brechungsindex, der jenem der Migrationsmatrix entspricht, durch den Schritt S7 des Einführens einer Nadel durch eine Eingangsöffnung der Migrationsvorrichtung in die Migrationsmatrix, so dass die Nadel durch die Matrix zumindest teilweise in die Abführungsstruktur zum Abführen von Fluiden eindringt.

Ein weiteres Ausführungsbeispiel der Erfindung betrifft ein Verfahren zur Migrationsanalyse, welches die Schritte S1 und S2, die oben beschrieben sind, umfasst. Außerdem beinhaltet das Verfahren den Schritt S8 des Einbringens einer Probenflüssigkeit in die Probenkammer, den Schritt S9 des Fixierens von Zellen der Probenflüssigkeit durch Einbringen einer Fixierflüssigkeit, den Schritt S10 des Färbens der Zellen durch Einbringen einer Färbeflüssigkeit in die Probenkammer und den Schritt S11 des optischen Vermessens einer Migrationsfähigkeit der Zellen, wobei sämtliche Schritte dieses Verfahrens mit derselben Migrationsvorrichtung durchgeführt werden.

In Fig. 12 ist ein Verfahren zur Migrationsanalyse gemäß einem weiteren Ausführungsbeispiel der Erfindung dargestellt. Das Verfahren umfasst den Schritt S20 der Migrations-Vorbereitung, den Schritt S21 der Migration, den Schritt S22 des Fixierens, den Schritt S23 des Lysierens, den Schritt S24 des Färbens, den Schritt S25 des Entfärbens des Hintergrunds, den Schritt S26 des Neutralisierens, den Schritt S27 des Trocknens und den Schritt S28 des optischen Vermessens, die im Folgenden näher beschrieben sind.

Bei der Migrationsanalyse mit der Migrationsvorrichtung, die beispielsweise in den Fig. 5A bis Fig. 5F dargestellt ist, kann im Detail wie folgt vorgegangen werden.

Der Schritt S20 der Migrations-Vorbereitung kann folgende Unterschritte aufweisen:
a) Entnahme der Migrationsvorrichtung 520 aufweisend das Oberteil 501, Unterteil 502, und die Matrices 503, 504 aus einer sterilen Verpackung;
b) Vorwärmen bzw. Temperieren der Migrationsvorrichtung 520 vorzugsweise auf 37°C in einem Wärmeschrank oder Inkubator.

Der Schritt S21 der Migration kann folgende Unterschritte aufweisen:
a) Abgabe einer definierten Teilmenge der verdünnten Probe 521 in die Probenkammern 512, optional können vor dem Auftragen mit verdünnter Probe die Matrices 503, 504 auch mit einer definierten Teilmenge der Pufferlösung vorbenetzt werden;
b) Abdeckung der Probenkammern 512 zwecks Minimierung des Verdunstungsvolumens bzw. Sättigung der relativen Luftfeuchte auf das Probenkammervolumen;
c) Rückführung der mit verdünnter Probe 521 gefüllten Migrationsvorrichtung 520 in den Wärmeschrank bzw. Inkubator für bis zu max. 60 Minuten, vorzugsweise 30 Minuten.

Der Schritt S22 des Fixierens kann folgende Unterschritte enthalten:
a) Nach der Inkubation (S21, Unterschritt c)), fixieren der Migrationszellen mit z.B. Formaldehyd durch Befüllen des Restvolumens der Probenkammer 512;
   Optional kann dieser Prozess auch zeitlich beschleunigt werden, indem nach kurzer Einwirkzeit die Probenkammer 512 entleert und nochmals mit Formaldehyd befüllt wird;
b) Entleeren der Probenkammer 512 vorzugsweise über die Klemmringdüsen 517, den Ringkanal 519 und den Fluidkanal 518 in den zentralen Absaugnippel 506.

Der Schritt S23 des Lysierens kann folgende Unterschritte aufweisen:
a) Lysieren der verbliebenen restlichen, nicht abgesaugten bzw. nicht bereits lysierten roten Blutkörperchen auf der Oberfläche der Migrationsmatrix 503 durch Befüllung der Probenkammern 512 mit z.B. entionisiertem Wasser oder Alternativ-Reagenzien und mehrminütigem Einwirken.
b) Entleeren
c) Tröpfchen-Wäschen: Auswaschen der mit Reagenz gefüllten Matrices 503, 504 und konstruktionsbedingten Fluidfilm auf der Oberfläche der Migrationsmatrix 503 mit entionisiertem Wasser und/oder tröpfchenweise dosierten Zwischen-Waschprozessen durch alternierendes Saugen von Luft und Wasser über die Klemmringdüsen 517 bzw. gleichzeitig über die Nut 505;
d) Zwischen-Trocknung: Kurzes Trocknen der Abführungsstrukturen 517, 519, 518 bzw. der Nut 505 sowie der Matrices 503, 504 durch Ansaugen mit vorzugweise angewärmter Luft.

Der Schritt S24 des Färbens (Staining) kann folgende Unterschritte aufweisen:
a) Befüllung der Probenkammern 512 mit z.B. Hämatoxylin und mehrminütigem Einwirken;
   optional kann der Unterschritt a) auch zweimal durchgeführt werden;
b) Entleeren;
c) Tröpfchen-Wäschen (Analog zu S23, Unterschritt c));
d) Zwischen-Trocknung (Analog zu S23, Unterschritt d)).

Der Schritt S25 der Entfärbung des Hintergrundes (Clearing) kann folgende Unterschritte enthalten:
a) Befüllung der Probenkammern 512 mit z.B. verdünnter HCI-Lösung und mehrminütigem Einwirken;
b) Entleeren;
c) Tröpfchen-Wäschen (Analog S23, Unterschritt c)).

Der Schritt S26 der Neutralisierung (Blueing) kann folgende Unterschritte enthalten:
a) Befüllung der Probenkammern 512 mit Neutralisierungspuffer (pH 9) und mehrminütigem Einwirken;
b) Entleeren;
c) Tröpfchen-Wäschen (Analog S23, Unterschritt c));
d) Optional: jeder dieser Prozesse (Lysieren, Färben, Entfärben, Neutralisieren mit anschließendem Entleeren, Tröpfchen-Waschen und Zwischen-Trocknen) kann auch, abhängig vom Prozesserfolg, mehrfach hintereinander durchgeführt werden.

Der Schritt S27 der Trocknung kann folgende Unterschritte enthalten:
a) Langzeit-Trocknen der Absaugstrukturen 517, 519, 518 bzw. der Nut 505 sowie der Matrices 503, 504 durch Ansaugen mit vorzugweise angewärmter Luft;
b) Entnahme der getrockneten Migrationsvorrichtung 520 aus dem Wärmeschrank oder Inkubator;
c) Lagerung der getrockneten Migrationsvorrichtung 520 bis zur Vermessung (max. 1 Jahr).

Der Schritt S28 der Optische Vermessung kann folgende Unterschritte enthalten:
Herstellung der Transparenz:
   a) Vorbereitung der Matrices 503, 504 zur optischen Vermessung durch Einbringen von Immersionsöl 515 mit einer schräg zulaufenden Nadel 507, z.B. einer Spritzennadel, indem der schräg zulaufende Teile der Nadelspitze beide Matrices im Bereich der Nut 505 des Unterbodens durchdringt und das Reagenz seitlich in und unter beide Matrices gleichzeitig eindringen kann (siehe Fig. 5F). Dieser Prozess kann durch Drehen der Nadel während der Einbringung und/oder gleichzeitiges Anlegen von Unterdruck an der Nut 505 noch verbessert werden;
Messung:
   b) Einlegen der Migrationsvorrichtung in ein 3D-Durchlicht-/Auflichtmikroskop;
   c) Optische Vermessung der in der Migrationsvorrichtung angeordneten Migrationsmatrix 503.

Ergänzend sei darauf hinzuweisen, dass "umfassend" oder "aufweisend" keine anderen Elemente ausschließt und "ein" oder "einer" keine Vielzahl ausschließt. Ferner sei darauf hingewiesen, dass Merkmale, die mit Verweis auf eines der obigen Ausführungsbeispiele oder Ausführungsformen beschrieben worden sind, auch in Kombination mit anderen Merkmalen anderer oben beschriebener Ausführungsbeispiele oder Ausführungsformen verwendet werden können. Bezugszeichen in den Ansprüchen betreffend die Vorrichtung oder das Verfahren sind nicht als Einschränkungen anzusehen.

## Patentansprüche

1. Migrationsvorrichtung (100, 520) zur Bestimmung der Migrationsfähigkeit amöboid beweglicher Zellen, aufweisend:
• eine in einem Gehäuse (101, 501, 502) angeordnete Probenkammer (102, 512) mit einer Eingangsöffnung zur Aufnahme einer Probenflüssigkeit enthaltend die amöboid beweglichen Zellen;
• mindestens eine auf einer Bodenfläche (202) der Probenkammer aufliegende, als dünne Schicht ausgebildete Migrationsmatrix (105, 503), in welche die amöboid beweglichen Zellen eindringen, wobei die mittlere Porengröße der Migrationsmatrix kleiner ist als der mittlere Durchmesser der amöboid beweglichen Zellen;
• mindestens einen Fluidauslass (103, 401, 402, 506);
• eine erste Abführungsstruktur (104, 201, 403, 518, 524), die in dem Gehäuse (101, 501, 502) ausgebildet ist und zum Abführen eines Fluides aus der Migrationsmatrix (105, 503) ausgeführt ist und eine in der Bodenfläche (202) angeordnete Abflussstruktur aufweist, und
• wobei die erste Abführungsstruktur in den zumindest einen Fluidauslass (103, 401, 402, 506) mündet.

2. Migrationsvorrichtung (100, 520) nach Anspruch 1, **gekennzeichnet durch** eine zweite Abführungsstruktur (301, 404, 519), die in dem Gehäuse (101, 501, 502) ausgebildet ist und zum Abführen eines Fluides aus der Probenkammer ausgeführt ist und zumindest eine Öffnung (301a, 404a, 513, 517) in einer Seitenwand der Probenkammer (102, 512) aufweist, wobei die zweite Abführungsstruktur in den zumindest einen Fluidauslass (103, 401, 402, 506) mündet und wobei die erste und die zweite Abführungsstruktur (301, 404, 519) dazu ausgeführt sind, die Abführung eines Fluides nach außerhalb des Gehäuses zu ermöglichen.

3. Migrationsvorrichtung (100, 520) nach Anspruch 1 oder 2, **dadurch gekennzeichnet, dass** die Migrationsmatrix (105, 503) unter Zwischenlage einer Depotmatrix (504) auf der Bodenfläche (202) aufliegt.

4. Migrationsvorrichtung (100, 520) nach einem der Ansprüche 1 bis 3, **dadurch gekennzeichnet, dass** die in der Bodenfläche ausgebildete Abführungsstruktur eine Nut (201a, 301a, 505) oder ein Nutsystem aufweist, die bzw. das einen Anteil von weniger als 20 %, vorzugsweise weniger als 10 %, der Bodenfläche einnimmt.

5. Migrationsvorrichtung (100, 520) nach einem der Ansprüche 1 bis 4, **dadurch gekennzeichnet, dass** die Abführungsstruktur einen in der Migrationsvorrichtung angeordneten, von der Probenkammer (102, 512) bis zum Fluidauslass (103, 402, 506) durchgehenden, ersten Fluidkanal (301, 404, 518) aufweist.

6. Migrationsvorrichtung (100, 520) nach einem der Ansprüche 2 bis 5, **dadurch gekennzeichnet, dass** die zweite Abführungsstruktur einen ringförmigen Fluidkanal (519) aufweist, wobei der ringförmige Fluidkanal (519) in der Migrationsvorrichtung um die Probenkammer (102, 512) herum angeordnet ist, wobei die zweite Abführungsstruktur eine Fluidverbindung zwischen dem ringförmigen Fluidkanal (519) und dem Fluidauslass aufweist; und wobei die zweite Abführungsstruktur eine Fluidverbindung zwischen den in der Seitenwand angeordneten Öffnungen (301a, 404a, 513, 517) und dem ringförmigen Fluidkanal (519) aufweist.

7. Migrationsvorrichtung (100, 520) nach einem der Ansprüche 2 bis 6, **dadurch gekennzeichnet, dass** die in der Bodenfläche ausgebildeten Teile der Abführungsstruktur und die in der Seitenwand angeordnete Öffnung (301a, 404a, 513, 517) mit demselben Fluidauslass verbunden sind, sowie dass die in der Bodenfläche ausgebildeten Teile der Abführungsstruktur und in die in der Seitenwand angeordnete Öffnung hinsichtlich ihrer Fluidverbindung seriell oder parallel geschaltet sind.

8. Migrationsvorrichtung (100, 520) nach einem der Ansprüche 2 bis 6, **dadurch gekennzeichnet, dass** die Migrationsvorrichtung zumindest zwei separate Fluidauslässe (401, 402) aufweist; und wobei die in der Bodenfläche ausgebildeten Teile der Abführungsstruktur und in die in der Seitenwand angeordnete Öffnung mit den zwei separaten Fluidauslässen verbunden sind.

9. Migrationsvorrichtung (520) nach einem der Ansprüche 2 bis 8, **gekennzeichnet durch:**
• ein Oberteil (501), aufweisend:
• eine von einer Seitenwand räumlich abgegrenzte Durchgangsöffnung;
• einen ersten Aufnahmebereich (509);
• ein Unterteil (502), aufweisend einen zweiten Aufnahmebereich (523);
• wobei das Oberteil (501) und das Unterteil (502) in einen montierten Zustand der Migrationsvorrichtung zusammenfügbar sind;
• wobei im montierten Zustand der Migrationsvorrichtung die Migrationsmatrix (503) im ersten Aufnahmebereich und im zweiten Aufnahmebereich angeordnet ist;
• wobei die Durchgangsöffnung mit der Seitenwand die Probenkammer (512) bildet und das Unterteil einen Boden der Probenkammer (512) ausbildet, wobei die Migrationsmatrix (503), vorzugsweise unter Zwischenlage der Depotmatrix (504), auf dem Boden angeordnet ist; und
• wobei das Unterteil Teile der Abführungsstruktur (505) aufweist.

10. Migrationsvorrichtung (520) nach Anspruch 9, **dadurch gekennzeichnet, dass** die Abführungsstruktur eine im Unterteil (502) angeordnete Nut (505) aufweist, wobei die Nut zumindest teilweise im zweiten Aufnahmebereich (523) angeordnet ist, so dass die Nut im montierten Zustand der Migrationsvorrichtung von der Migrationsmatrix (503), vorzugsweise unter Zwischenlage der Depotmatrix (504), abgedeckt ist.

11. Verwendung einer Migrationsvorrichtung (100, 520) nach einem der vorangehenden Ansprüche zum Bestimmen der Migrationsfähigkeit amöboid beweglicher Zellen.

12. Verfahren zum Betreiben einer Migrationsvorrichtung nach einem der Patentansprüche 1 bis 10, **gekennzeichnet durch** die Schritte:
• Bereitstellen einer Migrationsvorrichtung aufweisend eine Probenkammer zur Aufnahme einer Probenflüssigkeit enthaltend amöboid bewegliche Zellen, eine Migrationsmatrix und eine Abführungsstruktur zum Abführen eines Fluides aus der Migrationsvorrichtung zu einem Fluidauslass der Migrationsvorrichtung (S1); und
• Abführen eines Fluids aus der Probenkammer und/oder aus der Migrationsmatrix durch die Abführungsstruktur zum Fluidauslass (S2).

13. Verfahren nach Anspruch 12 zum Entfernen einer Probe oder einer Behandlungsflüssigkeit, **gekennzeichnet durch** den Schritt:
µ Einbringen der Probe oder der Behandlungsflüssigkeit in die Probenkammer (S3); und
µ wobei der Schritt des Abführens eines Fluides ein Abführen zumindest eines Teils der Probe beziehungsweise zumindest eines Teils der Behandlungsflüssigkeit durch die Abführungsstruktur umfasst.

14. Verfahren nach Anspruch 12 oder 13 zur Migrationsanalyse, zusätzlich **gekennzeichnet durch** die Schritte:
• Einbringen einer Probenflüssigkeit in die Probenkammer (S8);
• Fixieren von Zellen der Probenflüssigkeit durch Einbringen einer Fixierflüssigkeit (S9);
• Färben der Zellen durch Einbringen einer Färbeflüssigkeit in die Probenkammer (S10);
• Einbringen einer Flüssigkeit mit einem Brechungsindex, der dem Brechungsindex der Migrationsmatrix entspricht (S6);
• optisches Vermessen eines Migrationsprofils der Zellen in der Migrationsmatrix (S11);
• wobei sämtliche Schritte dieses Verfahrens in der Migrationsvorrichtung durchgeführt werden.

15. Verfahren nach Anspruch 14, wobei der Schritt des Einbringen einer Flüssigkeit mit einem Brechungsindex, der dem Brechungsindex der Migrationsmatrix entspricht (S6), durch Einführen einer Nadel durch eine Eingangsöffnung der Migrationsvorrichtung in die Migrationsmatrix erfolgt, so dass die Nadel durch die Migrationsmatrix zumindest teilweise in die Abführungsstruktur zum Abführen von Fluiden eindringt (S7).

## Claims

1. Migration device (100, 520) for determining the migratory ability of cells capable of amoeboid movement, comprising
• a sample chamber (102, 512) arranged in a housing (101, 501, 502) and having an inlet opening for receiving a sample liquid containing the cells capable of amoeboid movement;
• at least one migration matrix (105, 503) which rests on a base surface (202) of the sample chamber and is formed as a thin layer, into which the cells capable of amoeboid movement penetrate, wherein the mean pore size of the migration matrix is smaller than the mean diameter of the cells capable of amoeboid movement;
• at least one fluid outlet (103, 401, 402, 506);
• a first discharge structure (104, 201, 403, 518, 524) formed in said housing (101, 501, 502) and adapted to discharge a fluid from said migration matrix (105, 503) and having a drain structure arranged in said base surface (202); and
• wherein the first discharge structure opens into the at least one fluid outlet (103, 401, 402, 506).

2. Migration device (100, 520) according to claim 1, **characterised by** a second discharge structure (301, 404, 519) formed in the housing (101, 501, 502) and adapted to discharge a fluid from the sample chamber, and at least one opening (301a, 404a, 513, 517) in a side wall of the sample chamber (102, 512), wherein the second discharge structure opens into the at least one fluid outlet (103, 401, 402, 506), and wherein the first and second discharge structures (301, 404, 519) are adapted to allow the discharge of a fluid to the outside of the housing.

3. Migration device (100, 520) according to claim 1 or 2, **characterised in that** the migration matrix (105, 503) rests on the base surface (202) with the interposition of a deposit matrix (504).

4. Migration device (100, 520) according to one of claims 1 to 3, **characterised in that** the discharge structure formed in the base surface comprises a groove (201a, 301a, 505) or groove system which occupies a proportion of less than 20%, preferably less than 10%, of the base surface.

5. Migration device (100, 520) according to one of claims 1 to 4, **characterised in that** the discharge structure comprises a first fluid channel (301, 404, 518) arranged in the migration device and extending from the sample chamber (102, 512) to the fluid outlet (103, 402, 506).

6. Migration device (100, 520) according to one of claims 2 to 5, **characterised in that** the second discharge structure comprises an annular fluid channel (519), wherein the annular fluid channel (519) is arranged in the migration device around the sample chamber (102, 512), wherein the second discharge structure has a fluid connection between the annular fluid channel (519) and the fluid outlet; and wherein the second discharge structure has a fluid connection between the openings (301a, 404a, 513, 517) arranged in the side wall and the annular fluid channel (519).

7. Migration device (100, 520) according to one of claims 2 to 6, **characterised in that** the parts of the discharge structure formed in the base surface and the opening (301a, 404a, 513, 517) arranged in the side wall are connected to the same fluid outlet, and **in that** the parts of the discharge structure formed in the base surface and the opening arranged in the side wall are connected in series or in parallel in terms of their fluid connection.

8. Migration device (100, 520) according to one of claims 2 to 6, **characterised in that** the migration device comprises at least two separate fluid outlets (401, 402); and wherein the parts of the discharge structure formed in the base surface and the opening arranged in the side wall are connected to the two separate fluid outlets.

9. Migration device (520) according to one of claims 2 to 8, **characterised by**
• an upper part (501), having:
• a through-opening spatially delimited from a side wall;
• a first receiving region (509);
• a lower part (502) having a second receiving region (523);
• wherein the upper part (501) and the lower part (502) can be joined together in an assembled state of the migration device;
• wherein in the assembled state of the migration device the migration matrix (503) is arranged in the first receiving region and in the second receiving region;
• wherein the through-opening together with the side wall forms the sample chamber (512) and the lower part forms a base of the sample chamber (512), wherein the migration matrix (503) is arranged on the base, preferably with the interposition of the deposit matrix (504); and
• wherein the lower part has parts of the discharge structure (505).

10. Migration device (520) according to claim 9, **characterised in that** the discharge structure comprises a groove (505) arranged in the lower part (502), wherein the groove is arranged at least partially in the second receiving region (523), so that the groove is covered by the migration matrix (503), preferably with the interposition of the deposit matrix (504), in the assembled state of the migration device.

11. Use of a migration device (100, 520) according to one of the preceding claims for determining the migratory ability of cells capable of amoeboid movement.

12. Method for operating a migration device according to one of claims 1 to 10, **characterised by** the steps:
• providing a migration device having a sample chamber for receiving a sample liquid containing cells capable of amoeboid movement, a migration matrix and a discharge structure for discharging a fluid from the migration device to a fluid outlet of the migration device (S1); and
• discharging a fluid from the sample chamber and/or from the migration matrix through the discharge structure to the fluid outlet (S2).

13. Method according to claim 12 for removing a sample or a treatment liquid, **characterised by** the step:
• introducing the sample or the treatment liquid into the sample chamber (S3); and
• wherein the step of discharging a fluid comprises a discharge of at least a part of the sample or at least a part of the treatment liquid through the discharge structure.

14. Method according to claim 12 or 13 for migration analysis, additionally **characterised by** the steps:
• introducing a sample liquid into the sample chamber (S8);
• fixing of cells of the sample liquid by introducing a fixing liquid (S9);
• staining the cells by introducing a staining liquid into the sample chamber (S10);
• introducing a liquid having a refractive index corresponding to the refractive index of the migration matrix (S6);
• optically measuring a migration profile of the cells in the migration matrix (S11);
• wherein all steps of this method are performed in the migration device.

15. Method according to claim 14, wherein the step of introducing a liquid having a refractive index that corresponds to the refractive index of the migration matrix (S6) is performed by inserting a needle through an inlet opening of the migration device into the migration matrix, so that the needle penetrates through the migration matrix at least partially into the discharge structure for discharging fluids (S7).

## Revendications

1. Dispositif de migration (100, 520) pour déterminer l'aptitude à la migration de cellules mobiles amiboïdes comprenant
- une chambre à échantillon (102, 512) dans un boîtier (101, 501, 502) ayant un orifice d'entrée pour recevoir un liquide échantillon contenant des cellules mobiles amiboïdes,
- au moins une matrice de migration (105, 503) réalisée sous la forme d'une couche mince, placée sur une surface de fond (202) de la chambre à échantillon, et dans laquelle pénètrent les cellules mobiles amiboïdes, la taille moyenne des pores de la matrice de migration étant inférieure au diamètre moyen des cellules mobiles amiboïdes,
- au moins une sortie de fluide (103, 401, 402, 506)
- une première structure d'évacuation (104, 201, 403, 518, 524) réalisée dans le boîtier (101, 501, 502) et conçue pour évacuer un premier fluide de la matrice de migration (105, 503) et une structure d'écoulement réalisée dans la surface de fond (202) et
- la première structure d'évacuation débouchant dans au moins une sortie de fluide (103, 401, 402, 506).

2. Dispositif de migration (100, 520) selon la revendication 1, **caractérisé par**
une seconde structure d'évacuation (301, 404, 519) réalisée dans le boîtier (101, 501, 502) et conçue pour évacuer un fluide de la chambre à échantillon et au moins une ouverture (301a, 404a, 513, 517) dans la paroi latérale de la chambre à échantillon (102, 512), la seconde structure d'évacuation débouchant dans au moins une sortie de fluide (103, 401, 402, 506), la première et la seconde structure d'évacuation (301, 404, 519) étant conçues pour permettre l'évacuation d'un fluide vers l'extérieur du boîtier.

3. Dispositif de migration (100, 520) selon revendication 1 ou 2, **caractérisé en ce que**
la matrice de migration (105, 503) s'appuie sur la surface de fond (202) avec interposition d'une matrice de dépôt (504).

4. Dispositif de migration (100, 520) selon l'une des revendications 1 à 3,
**caractérisé en ce que**
la surface de fond a une rainure (201a, 301a, 505) ou un système de rainures occupant une fraction de la surface du fond inférieure à 20% et de préférence elle est inférieure à 10%.

5. Dispositif de migration (100, 520) selon l'une des revendications 1 à 4,
**caractérisé en ce que**
la structure d'évacuation comprend un premier canal de fluide (301, 404, 518) prévu dans le dispositif de migration et traversant de la chambre à échantillon (102, 512) jusqu'à la sortie de fluide (103, 402, 506).

6. Dispositif de migration (100, 520) selon l'une des revendications 2 à 5,
**caractérisé en ce que**
la seconde structure d'évacuation comporte un canal de fluide (519) de forme annulaire, ce canal de fluide annulaire (519) étant prévu dans le dispositif de migration autour de la chambre à échantillon (102, 512), la seconde structure d'évacuation comprenant une liaison de fluide entre le canal annulaire de fluide (519) et la sortie de fluide, la seconde structure d'évacuation réalisant une liaison fluidique entre les orifices (301a, 404a, 513, 517) dans la paroi latérale et le canal annulaire de fluide (519).

7. Dispositif de migration (100, 520) selon l'une des revendications 2 à 6,
**caractérisé en ce que**
les parties de la structure d'évacuation réalisées dans la surface de fond et l'orifice (301a, 401a, 513, 517) dans la paroi latérale sont reliées à la même sortie de fluide ainsi que les parties de la structure d'évacuation réalisées dans la surface de fond et l'orifice dans la paroi latérale étant branchées par une liaison fluidique en série ou en parallèle.

8. Dispositif de migration (100, 520) selon l'une des revendications 2 à 6,
**caractérisé en ce que**
le dispositif de migration comporte au moins deux sorties de fluide (401, 402) séparées et
les parties de la structure d'évacuation réalisées dans la surface de fond et l'orifice réalisé dans la paroi latérale sont reliés aux deux sorties de fluide, distinctes.

9. Dispositif de migration (520) selon l'une des revendications 2 à 8, **caractérisé par**
- une partie supérieure (501) ayant :
- un orifice traversant séparé dans l'espace de la paroi latérale,
- une première zone de réception (509)
- une partie inférieure (502) comprenant une seconde zone de réception (523),
- la partie supérieure (501) et la partie inférieure (502) étant réunies à l'état monté du dispositif de migration
- à l'état monté du dispositif de migration, la matrice de migration (503) se trouve dans la première zone de réception et dans la seconde zone de réception ;
- l'orifice traversant forme avec la paroi latérale, la chambre à échantillon (512) et la partie inférieure forme le fond de la chambre à échantillon (512),
la matrice de migration (503) étant prévue sur le fond, de préférence avec interposition de la matrice de dépôt (504) et
- la partie inférieure comporte des parties de la structure d'évacuation (505).

10. Dispositif de migration (520) selon la revendication 9,
**caractérisé en ce que**
la structure d'évacuation comporte une rainure (505) dans la partie inférieure (502), la rainure étant au moins en partie dans la seconde zone de réception (523) de sorte qu'à l'état monté du dispositif de migration, la rainure est couverte par la matrice de migration (503), de préférence avec interposition de la matrice de dépôt (504).

11. Utilisation d'un dispositif de migration (100, 520) selon l'une des revendications précédentes pour déterminer l'aptitude à la migration de cellules mobiles amiboïdes.

12. Procédé de gestion d'un dispositif de migration selon l'une des revendications 1 à 10,
**caractérisé par** les étapes consistant à :
- fournir (S1) un dispositif de migration comportant une chambre à échantillons pour recevoir un liquide échantillons contenant des cellules mobiles amiboïdes, une matrice de migration et une structure d'évacuation pour évacuer un liquide du dispositif de migration, pour la sortie de fluide du dispositif de migration ; et
- évacuer (S2) un fluide de la chambre à échantillons et/ou de la matrice de migration par la structure d'évacuation vers la sortie de fluide.

13. Procédé selon la revendication 12 pour éliminer un échantillon ou un liquide de traitement
**caractérisé par** les étapes consistant à :
- introduire (S3) l'échantillon ou le liquide de traitement dans la chambre à échantillons et
- l'étape d'évacuation d'un fluide consistant à évacuer au moins une partie de l'échantillon ou au moins une partie du liquide de traitement par la structure d'évacuation.

14. Procédé selon la revendication 12 ou 13 pour l'analyse de migration, **caractérisé en outre par** les étapes consistant à :
- introduire (S8) un liquide échantillon dans la chambre à échantillons
- fixer (S9) des cellules de liquide échantillon par l'introduction d'un liquide de fixation
- teinter (S10) les cellules en introduisant une teinte dans la chambre à échantillon
- introduire (S6) un liquide avec un indice de réfraction qui correspond à l'indice de réfraction de la matrice de migration
- faire (S11) une mesure optique du profil de migration des cellules dans la matrice de migration,
- toutes les étapes du procédé se faisant dans le dispositif de migration.

15. Procédé selon la revendication 14,
selon lequel
l'étape d'introduction d'un liquide avec un indice de réfraction qui correspondant à l'indice de réfraction de la matrice de migration (S6) se fait par l'introduction d'une aiguille à travers un orifice d'entrée du dispositif de migration dans la matrice de migration de façon que l'aiguille pénètre à travers la matrice de migration au moins en partie dans la structure d'évacuation pour évacuer le fluide (S7).
